# EUROPEAN PATENT SPECIFICATION

(11) **EP 0 412 404 B1**
(45) Date of publication and mention of the grant of the patent: **31.01.1996**
(21) Application number: 90114628.2
(22) Date of filing: 31.07.1990
(51) Int. Cl.: C07D 277/38, A61K 31/425, C07D 277/56, C07D 417/12

(54) **Thiazole derivatives, processes for production thereof and pharmaceutical compositions comprising the same**
Thiazolderivate, Verfahren zur Ihrer Herstellung und pharmazeutische Zusammensetzungen, die sie enthalten
Dérivés de thiazole, procédé et compositions pharmaceutiques les contenant

(30) Priority: 07.08.1989 GB 8918045; 21.02.1990 GB 9003930
(43) Date of publication of application: 13.02.1991
(73) Proprietor: FUJISAWA PHARMACEUTICAL CO., LTD., Osaka-shi Osaka 541 (JP)
(72) Inventor: Matsuo, Masaaki, Toyonaka-shi, Osaka 560 (JP); Ogino, Takashi, Kita-ku, Kobe-shi, Hyogo 651-11 (JP); Igari, Norihiro, Amagasaki-shi, Hyogo 660 (JP); Seno, Hachiro, Kadoma-shi, Osaka 571 (JP); Shimomura, Kyoichi, Suita-shi, Osaka 565 (JP)
(74) Representative: Türk, Gille, Hrabal, Leifert

(56) References cited:
- EP-A- 0 117 082
- FR-A- 1 321 897
- JOURNAL OF THE INDIAN CHEMICAL SOCIETY, vol. 39, no. 6, 1962, pages 420-426, Calcutta, IN; C.S. MAHAJANSHETTI et al.: "2-Aminothiazole derivatives. Part I"
- JOURNAL OF THE INDIAN CHEMICAL SOCIETY, vol. 40, no. 11, 1963, pages 921-924,
- Calcutta, IN; C.S. MAHAJANSHETTI et al.: "2-Aminothiazole derivatives. Part III"
- CHEMICAL ABSTRACTS, vol. 82, no. 13, 31st March 1975, page 93, abstract no. 81110d, Columbus, Ohio, US; C.S. MAHAJANSHETTI et al.: "2-Amino-4-methyl-5- thiazolyl phenyl sulfides and sulfones as possible antibacterials", & J. KARNATAK UNIV. 1973, 18, 5-10
- JOURNAL OF MEDICINAL CHEMISTRY, vol. 12, no. 5, May 1969, pages 357-363, Washington, DC, US; E.F. ELSLAGER et al.: "Repository drugs. IV. 4',4'''- sulfonylbisacetanilide (acedapsone, DADDS) and related sulfanilylanilides with prolonged antimalarial and antileprotic action"
- CANADIAN JOURNAL OF CHEMISTRY, vol. 45, no. 15, 1967, pages 1807-1810, CA; C.S. MAHAJANSHETTI et al.: "A facile nucleophilic displacement of bromine in 2- amino-4-methyl-5-bromothiazole by a thiophenoxide anion"
- ARZNEIMITTEL-FORSCHUNG, vol. 26, no. 8, August 1976, pages 1343-1547, Aulendorf, DE; I. HAGEDORN et al.: "Reaktivierung phosphorylierter Acetylcholinesterase (AChE): Quartäre Salze vinyloger Pyridinaldoxime"
- MONATSHEFTE FÜR CHEMIE, vol. 120, no. 11, November 1989, pages 991-995, Springer-Verlag, AT; M.M. EL-KERDAWY et al.: "Synthesis of substituted 4H- thiazolo[4,5-b][1]benzothiopyran-4-ones as possible schistosomicidal agents"

## Description

This invention relates to new thiazole derivatives.

More particularly, this invention relates to new thiazole derivatives and pharmaceutically acceptable salts thereof which have pharmacological activities, processes for production thereof, pharmaceutical compositions comprising the same and methods of use thereof.

EP-A-0 117 082 discloses thiazole derivatives which are useful as cardiotonic agents and anti-ulcer agents.

Arzneimittel-Forschung 1976, 26, 1543-47 discloses chemotherapeutically active nitrodiphenyl sulfones showing a good systemic activity against tubercle bacilli and plasmodia. However, the advantages of these compounds were insignificant as compared to diamino-diphenyl sulfone.

Accordingly, one object of this invention is to provide new and useful thiazole derivatives and pharmaceutically acceptable salts thereof.

Another object of this invention is to provide processes for production of said thiazole derivatives and pharmaceutically acceptable salts thereof.

A further object of this invention is to provide pharmaceutical compositions comprising said thiazole derivatives or pharmaceutically acceptable salts thereof.

Still further object of this invention is to provide methods of using said thiazole derivatives or pharmaceutically acceptable salts thereof for therapeutic treatment of rheumatism, nephritis, thrombocytopenia, tumor or side effect of an antitumor agent in human being and animals.

The object thiazole derivatives of this invention are novel and represented by the following general formula (I): 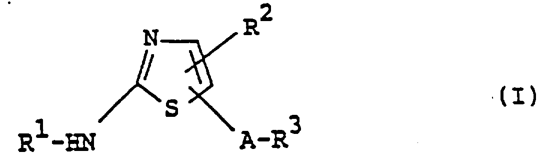 wherein
- R¹: is hydrogen (C₁-C₆)alkanoyl, (C₁-C₆)alkanesulfonyl or benzoyl which may be substituted with halogen,
- R: is hydrogen, (C₁-C₆) alkyl, hydroxy (C₁-C₆)alkyl, halogen or carboxy,
- A: is -C(=NOR⁴)- [wherein R⁴ is (C₁-C₆) alkyl], 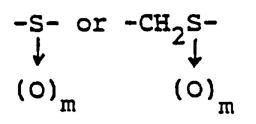 [wherein m is 0, 1 or 2], and
- R³: is phenyl, toluolyl, xylyl, cumenyl or naphthyl which may be substituted with halogen, hydroxy, (C₁-C₆)alkoxy, nitro, amino or acylamino; or
pyridyl, pyrimidinyl, imidazolyl, tetrazolyl or thiadiazolyl, each of which may be substituted with (C₁-C₆)alkyl, amino, hydroxy or halo(C₁-C₆)alkyl, provided that R³ is pyridyl, pyrimidinyl, imidazolyl, tetrazolyl or thiadiazolyl, each of which may be substituted with (C₁-C₆)alkyl, amino, hydroxy or halo(C₁-C₆)alkyl when A is 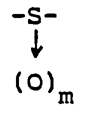 [wherein m is an integer of 0, 1, or 2]

The object compound (I) of the present invention can be prepared by the following processes.

### Process 1

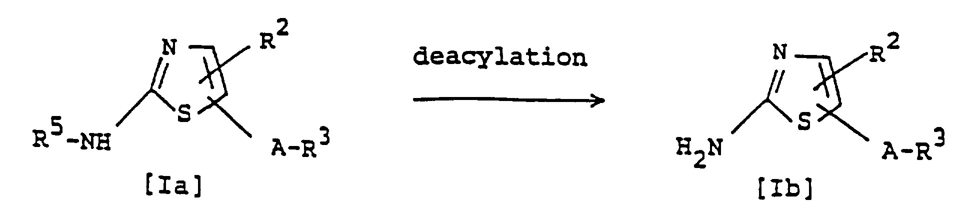

### Process 2

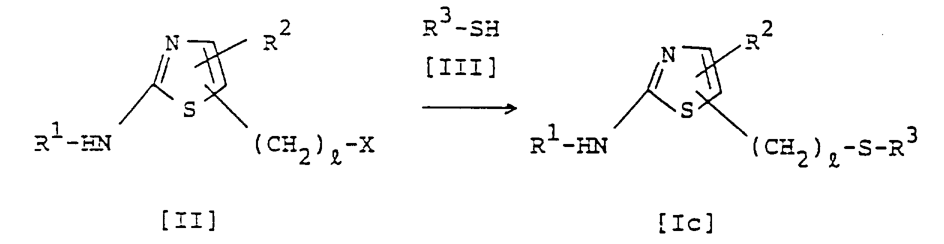

### Process 3

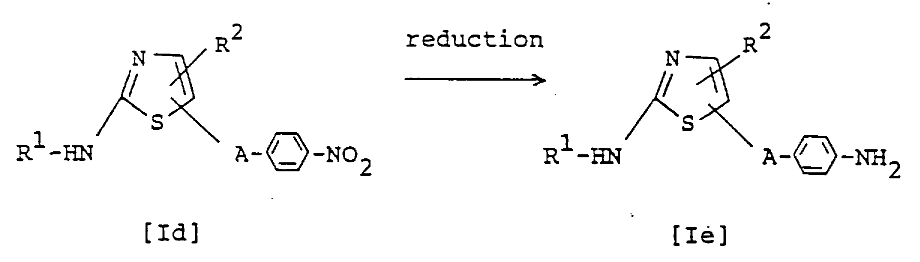

### Process 4

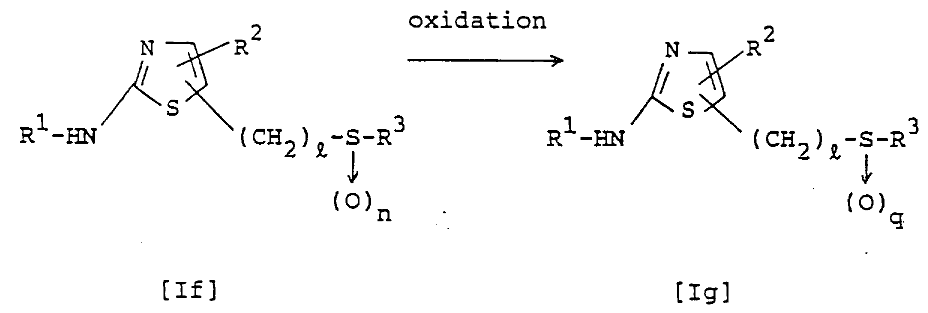

### Process 5

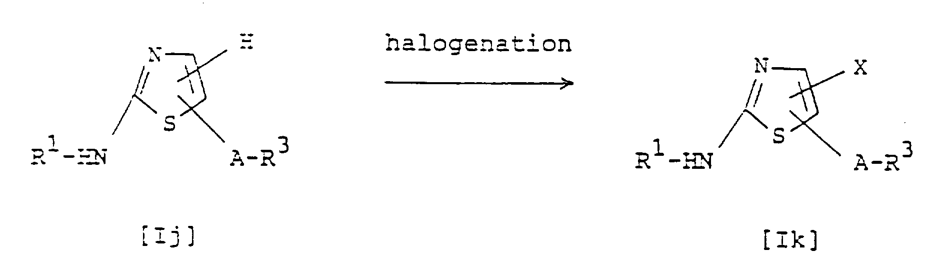

### Process 6

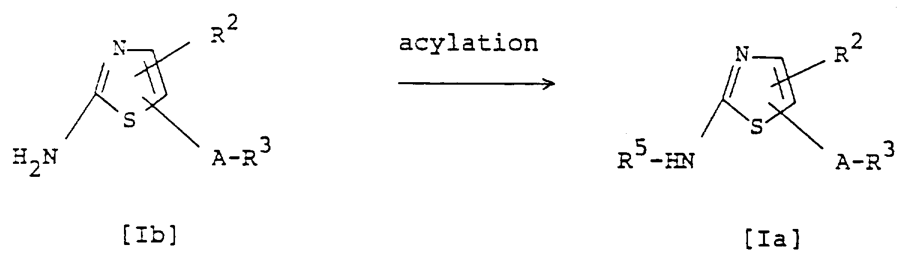

### Process 7

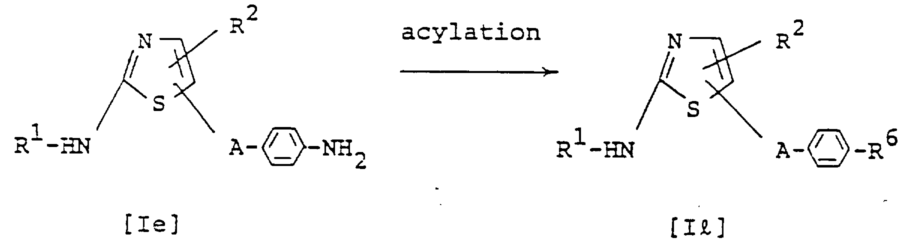

### Process 8

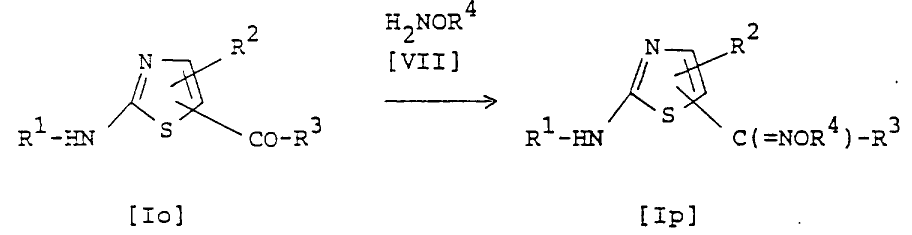

wherein
- R¹, R, R³, R⁴ and A: are each as defined above,
- R⁵: is acyl which may be substituted with halogen,
- R⁶: is acylamino,
- X: is halogen,
- ℓ: is 0 or 1,
- n: is 0 or 1 and
- q: is 1 or 2,
provided that q is 2 when n is 1.

A kind of the starting compounds [II] can be prepared by the process as illustrated in the following.

### Process A

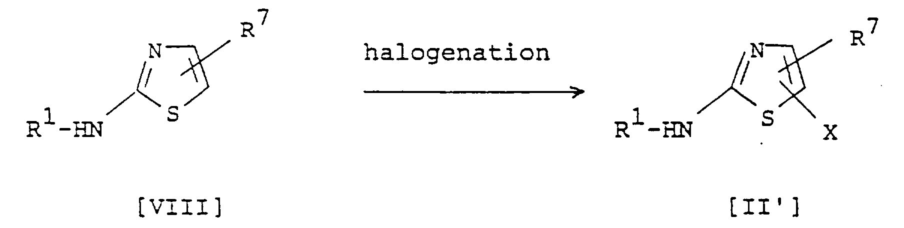

wherein
- R¹: and X are each as defined above, and
- R⁷: is (C₁-C₆)alkyl or hydroxy(C₁-C₆)alkyl.

In the above and subsequent of the present specification, suitable examples and illustrations for the various definitions to be included within the scope of the invention are explained in detail as follows:

Suitable "(C₁-C₆)alkyl" may be a straight or branched one such as methyl, ethyl, propyl, isopropyl, butyl, isobutyl, tert-butyl, pentyl, hexyl or the like.

Suitable example of "(C₁-C₆)alkyl" moiety in the term "hydroxy(C₁-C₆)alkyl" and "halo(C₁-C₆)alkyl" can be referred to the ones as exemplified above.

Suitable "halo(C₁-C₆)alkyl" may include "monohalo(C₁-C₆)alkyl" [e.g. chloromethyl, bromomethyl fluoromethyl, etc.], "dihalo(C₁-C₆)alkyl" [e.g. dichloromethyl, dibromomethyl, difluoromethyl, etc.] and "trihalo(C₁-C₆)alkyl" [e.g. trichloromethyl, tribromomethyl, trifluoromethyl, trifluoroethyl, etc.] and the like.

Suitable "(C₁-C₆)alkoxy" may include methoxy, ethoxy, propoxy, isopropoxy, butoxy, isobutoxy, t-butoxy, pentyloxy, hexyloxy and the like.

Suitable "halogen" may include fluorine, chlorine, bromine and iodine.

The (C₁-C₆)alkanoyl may include formyl, acetyl, propionyl, butyryl, isobutyryl, valeryl, isovaleryl, pivaloyl, hexanoyl, etc., (C₁-C₆)alkanesulfonyl may include methanesulfonyl, ethanesulfonyl, propanesulfonyl, butanesulfonyl, pentanesulfonyl, hexanesulfonyl, etc., and the like.

Suitable example of "acyl" moiety in the term of "acylamino" may include an aliphatic acyl, an aromatic acyl and an aliphatic acyl substituted with aromatic group(s).

The aliphatic acyl may include saturated or unsaturated, acyclic or cyclic ones, such as (C₁-C₆)alkanoyl (e.g. formyl, acetyl, propionyl, butyryl, isobutyryl, valeryl, isovaleryl, pivaloyl, hexanoyl, etc.), (C₁-C₆) alkoxycarbonyl (e.g. methoxycarbonyl, ethoxycarbonyl, propoxycarbonyl, butoxycarbonyl, tert-butoxycarbonyl, etc.), (C₁-C₆) alkanesulfonyl (e.g. methanesulfonyl, ethanesulfonyl, propanesulfonyl, butanesulfonyl, pentanesulfonyl, hexanesulfonyl, etc.), (C₁-C₆) alkenoyl (e.g. acryloyl, methacryloyl, crotonoyl, etc.), carbamoyl and the like.

The aromati acyl may include aroyl (e.g.benzoyl, toluoyl, xyloyl, etc.) and the like.

The aliphatic acyl substituted with aromatic group(s) may include ar(C₁-C₆)alkanoyl such as phenyl(C₁-C₆)alkanoyl (E.g. phenylacetyl, phenylpropionyl, phenylhexanoyl, etc.), ar(C₁-C₆)alkoxycarbonyl such as phenyl(C₁-C₆)alkoxycarbonyl (e.g. benzyloxycarbonyl, phenethyloxycarbonyl, etc.), phenoxy(C₁-C₆)alkanoyl (e.g. phenoxyacetyl, phenoxypropionyl, etc.), and the like.

Suitable pharmaceutically acceptable salts of the object compounds [I] are conventional non-toxic salts and include an organic acid salt [e.g. formate, acetate, trifluoroacetate, maleate, tartrate, methanesulfonate, benzenesulfonate, toluenesulfonate, etc.], an inorganic acid salt [e.g. hydrochloride, hydrobromide, sulfate, phosphate, etc.], a salt with an amino acid [e.g. arginine, glutamic acid, ornithine, etc.], a metal salt such as an alkali metal salt [e.g. sodium salt, potassium salt, etc.] and an alkaline earth metal salt [e.g. calcium salt, magnesium salt, etc.], an ammonium salt, an organic base salt [e.g. trimethylamine salt, triethylamine salt, pyridine salt, picoline salt, dicyclohexylamine salt, N,N'-dibenzylethylenediamine salt, etc.], and the like.

In this respect, it is to be noted that the compounds [Ia] to [Ip] are included within the scope of the compound [I], and accordingly the suitable salts of these compounds [Ia] to [Ip] are to be referred to those as exemplified for the object compounds [I] in the above.

The processes for preparing the object compound [I] or salts thereof are explained in detail in the following.

### Process 1

The object compound [Ib] or its salt can be prepared by deacylating a compound [Ia] or its salt.

Suitable method for this deacylation reaction may include conventional one such as hydrolysis and the like.

Hydrolysis is preferably carried out in the presence of an acid.

Suitable acid may be an inorganic acid [e.g. hydrochloric acid, hydrobromic acid, sulfuric acid, etc.], an organic acid [e.g. formic acid, acetic acid, trifluoroacetic acid, propionic acid, benzenesulfonic acid, p-toluenesulfonic acid, etc.], an acidic ion-exchange resin and the like. In case that the organic acid such as trifluoroacetic acid and p-toluenesulfonic acid is used in this reaction, the reaction is preferably carried out in the presence of cation trapping agents [e.g. anisole, etc.].

The acid suitable for this hydrolysis can be selected according to the kinds of the acyl group to be removed.

The hydrolysis is usually carried out in a conventional solvent which does not adversely influence the reaction such as water, methanol, ethanol, propanol, tert-butyl alcohol, tetrahydrofuran, N,N-dimethylformamide, dioxane or a mixture thereof, and further the above-mentioned acids can also be used as a solvent when they are in liquid.

The reaction temperature of this hydrolysis is not critical, and the reaction is usually carried out under cooling, at ambient temperature or under heating.

In this process, when the starting compound [Ia] or its salt has a acylamino group for R³, the group is also converted to an amino group.

### Process 2

The object compound [Ic] or its salt can be prepared by reacting a compound [II] or its salt with a compound [III] or its salt.

Suitable salts of the compound [II] and [III] may be the same as those exemplified as base salts of the object compound [I].

This reaction is usually carried out in a solvent such as methanol, ethanol, propanol, tetrahydrofuran, dioxane, dimethylformamide or any other organic solvent which does not adversely influence the reaction.

In case that a free form of the compound [III] is used in this reaction, the reaction is preferably carried out in the presence of a conventional base, such as alkali metal hydride [e.g. sodium hydride, potassium hydride, etc.], alkaline earth metal hydride [e.g. calcium hydride, magnesium hydride, etc.], alkali metal hydroxide [e.g. sodium hydroxide, potassium hydroxide, etc.], alkali metal carbonate [e.g. sodium carbonate, potassium carbonate, etc.], alkali metal bicarbonate [e.g. sodium bicarbonate, potassium bicarbonate, etc.], alkali metal fluoride [e.g. potassium fluoride, cesium fluoride, etc.], alkali metal alkoxide [e.g. sodium methoxide, sodium ethoxide, potassium tert-butoxide, etc.], trialkylamine [e.g. trimethylamine, triethylamine, etc.], picoline, 1,5-diazabicyclo[4,3,0]non-5-ene, 1,4-diazabicyclo[2,2,2]octane, 1,5-diazabicyclo[5,4,0]undecene-5 or the like.

The reaction temperature is not critical, and the reaction is usually carried out at ambient temperature or under cooling, warming or heating.

### Process 3

The object compound [Ie] or its salt can be prepared by reducing a compound [Id] or its salt.

The reduction can be carried out in a conventional manner, namely, chemical reduction or catalytic reduction.

Suitable reducing-agents to be used in chemical reduction are a combination of metal (e.g. stannum, zinc, iron, etc.) and ammonium chloride or an base (e.g. ammonia, sodium hydroxide, etc.), a combination of metal (e.g. tin, zinc, iron, etc.) or metallic compound (e.g. chromium chloride, stannous chloride, chromium acetate, etc.) and an organic or inorganic acid (e.g. formic acid, acetic acid, propionic acid, trifluoroacetic acid, p-toluenesulfonic acid, hydrochloric acid, hydrobromic acid, etc.), alkali metal borohydride (e.g. lithium borohydride, sodium borohydride, potassium borohydride, etc.) alkali metal cyanoborohydride (e.g. sodium cyanoborohydride, etc.) or alkali metal aluminium hydride (e.g. lithium aluminum hydride, etc.) the like.

Suitable catalysts to be used in catalytic reduction are conventional ones such as platinum catalyst (e.g. platinum plate, spongy platinum, platinum black, colloidal platinum, platinum oxide, platinum wire, etc.), palladium catalyst (e.g. palladium on carbon, colloidal palladium, palladium on barium sulfate, palladium on barium carbonate, etc.), nickel catalyst (e.g. reduced nickel, nickel oxide, Raney nickel, etc.), cobalt catalyst (e.g. reduced cobalt, Raney cobalt, etc.), iron catalyst (e.g. reduced iron, Raney iron, etc.), copper catalyst (e.g. reduced copper, Raney copper, Ullman copper, etc.) or the like.

The reduction of this process is usually carried out in a solvent such as water, alcohol (e.g. methanol, ethanol, propanol, etc.), acetic acid, dioxane, tetrahydrofuran, N,N-dimethylformamide, dimethyl sulfoxide, or any other organic solvent which does not adversely influence the reaction, or a mixture thereof. In case above-mentioned reducing agent is liquid, it can be also used as a solvent.

The reaction is preferably carried out under cooling to warming or heating.

### Process 4

A compound [Ig] or its salt can be prepared by subjecting a compound [If] or its salt to oxidation.

Oxidation in this process is carried out in a conventional manner with a conventional oxidizing agent which can oxidize a -S- group into -SO- or -SO₂- group, or -SO- group into -SO₂- group.

Suitable example of such oxidizing agent are inorganic peracid or its salt (e.g. periodic acid, persulfuric acid, etc.) or the sodium or potassium salt thereof, an organic peracid or its salt (e.g. perbenzoic acid, 3-chloroperbenzoic acid, performic acid, peracetic acid, chloroperacetic acid, trifluoroperacetic acid, etc. or the sodium or potassium salt thereof, etc.), ozone, hydrogen peroxide, urea-hydrogen peroxide and the like.

The present reaction is preferably conducted in the presence of a compound comprising a Group Vb or VIb metal in the Periodic Table, for example, tungstic acid, molybdic acid, vanadic acid, etc. or their salt with an alkali metal (e.g. sodium, potassium, etc.), an alkaline earth metal (e.g. calcium, magnesium, etc.) or ammonium, etc., or vanadium pentoxide.

The present oxidation is usually conducted in a solvent such as water, acetic acid, ethyl acetate, chloroform, dichloromethane, tetrahydrofuran, dioxane, N,N-dimethylformamide or any other solvent which does not give bad influence to the present reaction.

There is not particular limitation to the reaction temperature, and the present reaction is usually conducted at ambient temperature or under cooling.

### Process 5

The object compound [Ik] or its salt can be prepared by halogenating a compound [Ij] or its salt.

Suitable halogenating agent of this reaction may include conventional ones for example, halogen [e.g. chlorine, bromine, iodine, etc.], sulfuryl halide [e.g. sulfuryl chloride, sulfuryl bromide, etc.], N-halosuccinimide [e.g. N-chlorosuccinimide, N-bromosuccinimide, etc.], pyridinium hydrohalide perhalide [e.g. pyridinium hydrobromide perbromide, pyridinium hydrochloride perchloride, etc.], quarternary ammonium perhalide [e.g. phenyltrimethylammonium perbromide, etc.], ω-trihaloacetophenone [e.g. ω-tribromoacetophenone, etc.], cupric or potassium bromide, selenium oxychloride, or the like. These halogenating agents may be selected according to the kind of the starting compound [Ij] to be used.

This reaction is usually carried out in a conventional solvent such as chloroform, methylene chloride, carbon tetrachloride, acetic acid, a mixture of hydrogen halide [e.g. hydrogen bromide, hydrogen chloride, etc.] and acetic acid, water, dimethylformamide or the like.

The reaction temperature is not critical, and the reaction is usually carried out under cooling, at ambient temperature or under warming or heating.

### Process 6

The object compound [Ia] or its salt can be prepared by acylating a compound [Ib] or its reactive derivatives at the amino group or a salt thereof.

Suitable reactive derivatives at the amino group of the compound [Ib] include conventional ones used in amidation for example, Schiff's base type imino or its tautomeric enamine type isomer formed by reaction of a compound [Ib] with a carbonyl compound, a silyl derivative formed by reaction of a compound [Ib] with a silyl compound such as trimethylsilylacetamide, bis(trimethylsilyl)acetamide or the like, a derivative formed by reaction of a compound [Ib] with phosphorus trichloride or phosgene, and the like.

Suitable acylating agent to be used in this reaction includes an organic acid such as alkanoic acid [e.g. formic acid, acetic acid, propionic acid, etc.], arenecarboxylic acid (e.g. benzoic acid, toluenecarboxylic acid, etc.) which may have halogen, lower alkanesulfonic acid [e.g. methanesulfonic acid, etc.], arylisocyanate [e.g. phenylisocyanate, etc.] which may have halogen and their reactive derivative.

The suitable reactive derivative may be a conventional one such as an acid halide [e.g. acid chloride, acid bromide, etc.], an acid azide, an acid anhydride, an activated amide, an activated ester and the like. When free acid is used as an acylating agent, the acylation reaction may preferably be conducted in the presence of a conventional condensing agent such as N,N'-dicyclohexylcarbodiimide or the like.

This reaction is usually carried out in a solvent which does not adversely influence the reaction such as water, tetrahydrofuran, chloroform, dioxane, pyridine, methylene chloride, N,N-dimethylformamide or the like.

The reaction temperature is not critical and the reaction can be carried out at any temperature under cooling to heating.

### Process 7

The object compound [Iℓ] or its salt can be prepared by acylating a compound [Ie] or its reactive derivativesat the amino group or a salt thereof.

Suitable reactive derivatives at the amino group are to be referred to those as exemplified in Process 6.

This reaction may be carried out in substantially the same manner as Process 6, and therefore the reaction mode and reaction conditions [e.g. acylating agent, solvent, reaction temperature, etc.] of this reaction are to be referred to those as explained in Process 6.

### Process 8

The object compound [Ip] or its salt can be prepared by reacting a compound [Io] or its salt with a hydroxylamine derivative [VII] or its salt.

A suitable salt of a hydroxylamine derivative [VII] may be a hydrohalide (e.g. hydrochloride, etc.).

This reaction is usually carried out in a conventional solvent such as water, alcohol (e.g. methanol, ethanol, propanol, etc.), tetrahydrofuran, dioxane, ethyl acetate, N,N-dimethylformamide, dimethyl sulfoxide or any other organic solvent which does not adversely influence the reaction. In case the compound [VII] is liquid, it can be also used as a solvent.

The reaction temperature is not critical, and the reaction can be carried out under cooling to warming or heating.

The process for preparing the starting compound [II] or its salt is explained in detail in the following.

### Process A

The compound [II'] or its salt can be prepared by halogenating a compound [VIII] or its salt.

Suitable salts of the compounds [II'] and [VIII] may be the same as those exemplified for the object compound [I].

This reaction may be carried out in substantially the same manner as Process 6, and therefore the reaction mode and reaction conditions [e.g. halogenating agent, solvent, reaction temperature, etc.] of this reaction are to be referred to those as explained in Process 6.

The object compounds and pharmaceutically acceptable salts thereof are novel and exhibit pharmacological activities and are useful for the treatment and prophylaxis of rheumatism (e.g. rheumarthritis, etc.), nephritis, thrombocytopenia [e.g. idiopathic thrombocytopenic purpura, secondary thrombocytopenic purpura, thrombocytopenia due to a side effect of an antitumor agent (e.g. mitomycin C, etc.) etc.], tumor, side effect of an antitumor agent (e.g. decrease of body weight, etc.) and the like.

In order to show the utility of the object compounds [I], antirheumatic, anti-nephritic and platelet number-increasing activities and alleviating activity for side effect of antitumor agent of the object compounds [I] are explained in the following.

### Antirheumatic activity

### Test 1 Effect on collagen induced arthritis in mice

### Method :

Ten male DBA/1 mice were used per group. Type II bovine collagen was dissolved in 0.1M acetic acid and emulsified in complete Freund's adjuvant (CFA). Mice were primed with 125 µg of Type II collagen in CFA intradermally at the base of the tail. Mice were challenged after 21 day with the same procedure. From the day of challenge, drug was administered orally once a day for 3 weeks and mice were inspected weekly for visual signs of arthritis. To evaluate the effect of drugs, an arthritic index was used. Arthritic index was obtained by scoring each limb 0 - 3 severity, ep sen ng join swelling and erythema (Grade 1), visible join disorder (Grade 2) and detectable join ankylosis (Grade 3), and by summing up scores of four limbs.

### Results :

| Compounds | Dose level (mg/kg) | Inhibition (%) |
|---|---|---|
| a compound of | | |
| Example 11 | 100 | 51 |
| Example 14 | 100 | 57 |
| Example 15 | 100 | 35 |
| Example 18 | 100 | 44 |
| Example 21 | 100 | 31 |

### Anti-nephritic activity

### Test 2 Effect on chloric GVH disease (nephritis)

### Method :

Six weeks old female (57BL/6 x DBA/2)F₁ and DBA/2 mice were used. Graft-versus-host (GVH) disease was induced in (57BL/6 x DBA/2)F₁ mice with two injections of DBA/2 spleen cells given 5 days apart. Each injection contained 5 x 10⁷ cells. From 3 days after second cell injection, drug was administered orally once a day for 8 weeks. To assess the renal disease, at 8 weeks after last cell injection, proteinuria were measured. The concentration of serum albumin in the urine was determined by the single radial immunodiffusion method using rabbit anti-mouse serum albumin antiserum. Ten mice were used per group. Antinephritic activity of the compound was expressed the inhibition of albumin in urine.

### Results :

| Compounds | Dose level (mg/kg) | Inhibition of albumin in urine (%) |
|---|---|---|
| a compound of | | |
| Example 11 | 100 | 96 |
| Example 14 | 100 | 90 |
| Example 15 | 100 | 98 |
| Example 18 | 100 | 70 |
| Example 20 | 100 | 74 |
| Example 21 | 100 | 78 |

### Platelet number-increasing activity

### Test 3 Increasing effect on the platelet number decreased by mitomycin C

### Method :

A test compound was given orally once a day for 5 days to male ddY mice aged 6 or 7 weeks. The animals were used in groups of 10. Mitomycin C (hereinafter referred to as MMC) at a dose of 3.2 mg/kg was given intravenously to mice on day 0, 2 and 4 after the initial dosing with the test compound. The number of platelets were counted 5 days after the final dosing with the test compound, in which mice were bled from the orbital plexus and the platelets were counted with an automatic blood analyzer. The number of platelets of each group was calculated on the basis of the number of platelets (%) obtained from the non-test compound group.

### Results :

| Compounds | Dose level (mg/kg) | Number of platelets (%) |
|---|---|---|
| a compound of | | |
| Example 8 | 32 | 164 |
| Example 9 | 32 | 150 |
| Example 15 | 32 | 184 |
| Example 16 | 32 | 154 |
| Example 18 | 32 | 210 |
| Example 42 | 32 | 135 |

### Alleviating activity for side effect of antitumor agent

### Test 4 Restoring effect on the body weight decreased by MMC

### Method :

A test compound was given orally once a day for 5 days to male ddY mice aged 6 or 7 weeks. The animals were used in groups of 10. MMC at a dose of 3.2 mg/kg was given intravenously to mice on day 0, 2 and 4 after the initial dosing with the test compound. The body weight of mice were measured on day 0 and day 8.

The body weight of non-test compound group which was only administered MMC as above was measured on day 0 and day 8 as a control.

### Results:

| | Dose of test compound (mg/kg) | Body weight (g) | |
|---|---|---|---|
| | | day 0 | day 8 |
| MMC & a compound of Example 15 | 100 | 32.7 | 31.1 |
| MMC & a compound of Example 16 | 100 | 32.6 | 31.1 |
| MMC & a compound of Example 42 | 100 | 32.6 | 30.2 |
| MMC (no test compound) | - | 32.6 | 28.6 |
| | | (mean of 10 mice) | |

For therapeutic administration, the object compounds [I] and pharmaceutically acceptable salts thereof are used in the form of conventional pharmaceutical composition such as powders, fine granules, granules, tablets, dragee, microcapsules, capsules, suppository, solution, suspension, emulsion, syrups and the like. If desired, diluents or disintegrators (e.g. sucrose, lactose, starch, crystalline cellulose, low-substituted hydroxypropyl cellulose, synthetic aluminum silicate, etc.), binding agents (e.g. cellulose, methylcellulose, hydroxypropylcellulose, hydroxypropylmethylcellulose, polypropylpyrrolidone, polyvinylpyrrolidone, gelatin, gum arabic, polyethyleneglycol, etc.), coloring agents, sweeting agents, lubricant (e.g. magnesium stearate, etc.) or the like, may be dispensed with said composition.

The dosage of said composition according to this invention depends on the patient's age, body weight, condition, etc., and it is generally administered by the oral route at the daily dose level of 1 mg to 1 g as the object compound [I] or a salt thereof, preferably 10 mg to 100 mg on the same basis, at the interval of 1 to 3 times a day. Typical unit doses may be 5 mg, 10 mg, 20 mg, 50 mg, 100 mg and the like, although these are only examples and not limitative, of course.

The following Preparations and Examples are given for the purpose of illustrating the present invention in more detail.

### Preparation 1

A mixture of 2-acetylamino-4-hydroxymethylthiazole (7.0 g) and N-chlorosuccinimide (6.5 g) in acetic acid (70 ml) was heated at 40°C for 3.5 hours with stirring. The reaction mixture was concentrated under reduced pressure and the residue was added the aqueous sodium bicarbonate. The mixture was extracted with a mixture of ethyl acetate and tetrahydrofuran (1:1), washed with water and dried over magnesium sulfate. The solvent was concentrated under reduced pressure and the residue was triturated with isopropyl ether. The precipitates were collected by filtration, washed with isopropyl ether and dried in vacuo to give 2-acetylamino-5-chloro-4-hydroxymethylthiazole (7.3 g, yield : 78.5%).
mp : 145-146°C
IR (Nujol) : 3150, 1690, 1550, 1285 cm⁻¹
NMR (DMSO-d₆, 60MHZ, ppm) : 2.17 (3H, s), 4.17 (2H, d, J=5Hz), 5.17 (1H, t, J=5Hz)
Mass : M⁺ 208, M⁺¹ 207, M 206, m/e 164, 147, 135

### Preparation 2

A mixture of 2-amino-4-methylthiazole hydrochloride (1.5 g) and N-chlorosuccinimide (1.6 g) in acetic acid (15 ml) was heated 40°C for 5.5 hours with stirring. The reaction mixture was poured into ice water and the solution was adjusted to pH 8.5 using sodium bicarbonate. The mixture was extracted with a mixture of tetrahydrofuran and ethyl acetate (1:1), washed with aqueous saturated sodium chloride and dried over magnesium sulfate. The solvent was concentrated under reduced pressure to give 2-amino-5-chloro-4-methylthiazole (1.4 g, yield: 94.6%, oil).
NMR (DMSO-d₆, 200MHZ, ppm) : 2.09 (3H, s), 7.00 (2H, br s)
Mass : M⁺ 150, M⁺¹ 149, M 148, m/e 133, 113, 99

### Preparation 3

To a solution of 2-amino-4-methylthiazole hydrochloride (3.0 g) in acetic acid (20 ml) was added once N-bromosuccinimide (4.0 g) at room temperature with stirring. The mixture was stirred at room temperature for 1.5 hours and the reaction mixture was poured into isopropyl ether under ice cooling. The precipitates were collected by filtration, washed with ethyl ether and dried in vacuo to give 2-amino-5-bromo-4-methylthiazole hydrochloride (4.1 g, yield : 89.1%).
mp : 175-178°C (dec.)
IR (Nujol) : 3200, 2500-2700, 1635 cm⁻¹
NMR (DMSO-d₆, 200MHZ, ppm) : 2.14 (3H, s), 8.90 (3H, br s)
Mass : M⁺³ 196, M⁺ 195, M⁺¹ 194, M 193, m/e 192, 191, 149, 123, 113

### Example 1

A mixture of 2-amino-4-(4-aminobenzoyl)thiazole (6 g) and methoxyamine hydrochloride (13 g) in methanol (800 ml) was stirred at room temperature for 16 hours. The reaction mixture was concentrated under reduced pressure and then to the residue was added water. The solution was adjusted to pH 8.5 using 10% aqueous sodium bicarbonate under ice-cooling. The precipitates were collected by filtration, washed with water and recrystallized from ethanol to give 2-amino-4-[(4-aminophenyl)methoxyiminomethyl]thiazole (4.9 g, yield : 72.1%).
mp : 181-183°C
IR (Nujol) : 3350, 3100, 1605, 1510, 1380 cm⁻¹
NMR (DMSO-d₆, 60MHZ, ppm) : 3.73 (3H, s), 5.29 (2H, s), 6.46 (2H, d, J=9Hz), 6.95 (1H, s), 7.00 (2H, d, J=9Hz)
Mass : M⁺¹ 249, M 248, m/e 217, 203

### Example 2

A mixture of 2-acetylamino-4-chloromethylthiazole (1.9 g), 4-nitrothiophenol (1.6 g) and potassium carbonate (2.0 g) in a N,N-dimethylformamide (50 ml) was heated at 100°C for 3 hours with- stirring. The reaction mixture was concentrated under reduced pressure and the residue was triturated with water. The precipitates were collected by filtration, washed with water and dried in vacuo to give 2-acetylamino-4-(4-nitrophenylthiomethyl)thiazole (2.95 g, yield : 95.5%).
mp : 165-166°C
IR (Nujol) : 3150, 1655, 1595, 1545, 1500, 1335, 1290 cm⁻¹
NMR (DMSO-d₆, 60MHZ, ppm) : 2.17 (3H, s), 4.67 (2H, s), 7.15 (1H, s), 7.60 (2H, d, J=8Hz), 8.17 (2H, d, J=8Hz)
Mass : M⁺¹ 310, M 309, m/e 267, 246, 155, 124, 113

### Example 3

To a mixture of 2-acetylamino-4-(4-nitrophenylthiomethyl)thiazole (11 g) and ammonium chloride (2 g) in a mixture of tetrahydrofuran (200 ml), ethanol (200 ml) and water (100 ml) was added portionwise the iron powder (17 g) at 80°C with stirring. The mixture was refluxed for 3 hours with stirring. The reaction mixture was filtered by suction and the filtrate was concentrated under reduced pressure and then the residue was triturated with water. The precipitates were collected by filtration, washed with water and dried in vacuo to give 2-acetylamino-4-(4-aminophenylthiomethyl)thiazole (9.3 g, yield : 93.6%).
IR (Nujol) : 3400, 3250, 3150, 1690, 1545, 1370, 1220 cm⁻¹
NMR (DMSO-d₆, 90MHZ, ppm) : 2.10 (3H, s), 3.90 (2H, s), 5.20 (2H, s), 6.50 (2H, d, J=8Hz), 6.70 (1H, s), 7.05 (2H, d, J=8Hz), 12.10 (1H, s)
Mass : M⁺¹ 280, M 279, m/e 236, 220, 216, 205

### Example 4

To a solution of 2-acetylamino-4-(4-aminophenylthiomethyl)thiazole (9.0 g) in ethyl acetate (300 ml) was added portionwise the 3-chloroperbenzoic acid (17 g) at 5°C with stirring. The mixture was stirred at room temperature for 16 hours. The reaction mixture was washed with aqueous sodium bicarbonate and dried over magnesium sulfate. The solvent was concentrated under reduced pressure to give solid. The solid was subjected to column chromatography on silica gel (silica gel 60, 70-230 mesh; Merck : 300 g) and eluted with a mixture of chloroform and methanol (10:1). The fractions containing the objective compound were combined and concentrated under reduced pressure to give 2-acetylamino-4-(4-aminophenylsulfonylmethyl)thiazole (4.85 g, yield: 48.3%).
mp : 135-137°C
IR (Nujol) : 3450, 3350, 3200, 1680, 1635, 1595, 1550, 1300 cm⁻¹
NMR (DMSO-d₆, 60MHZ, ppm) : 2.17 (3H, s), 4.50 (2H, s), 6.17 (2H, s), 6.63 (2H, d, J=8Hz), 6.90 (1H, s), 7.35 (2H, d, J=8Hz)

### Example 5

A solution of 2-acetylamino-4-(4-aminophenylsulfonylmethyl)thiazole (4.8 g) in a mixture of acetic acid (35 ml) and 6N-aqueous hydrochloric acid (10 ml) was refluxed for 2.5 hours with stirring. The reaction mixture was poured into ice-water and then the solution was adjusted to pH 8.0 using 10% aqueous sodium bicarbonate with stirring. The precipitates were collected by filtration, washed with water and dried in vacuo to give 2-amino-4-(4-aminophenylsulfonylmethyl)-thiazole (2.50 g, yield : 60.2%).
mp : 203-206°C (dec.)
IR (Nujol) : 3450, 3350, 1630, 1595, 1530 1380 cm⁻¹
NMR (DMSO-d₆, 60MHZ, ppm) : 4.20 (2H, s), 6.03 (2H, s), 6.27 (1H, s), 6.57 (2H, d, J=8Hz), 6.85 (2H, s), 7.33 (2H, d, J=8Hz)
Mass : M 269, m/e 205, 162, 140, 113

### Example 6

A mixture of 4-nitrothiophenol (9.3 g), 2-amino-4-chloromethylthiazole hydrochloride (11 g) and potassium carbonate (20 g) in N,N-dimethylformamide (200 ml) was heated at 85-90°C for 5 hours with stirring. The reaction mixture was concentrated under reduced pressure and the residue was triturated with water. The precipitates were collected by filtration, washed with water and dried in vacuo to give 2-amino-4-(4-nitrophenylthiomethyl)thiazole (15.80 g, yield : 98.6%).
IR (Nujol) : 3400, 3100, 1630, 1530, 1340 cm⁻¹
NMR (DMSO-d₆, 60MHZ, ppm) : 4.23 (2H, s), 6.60 (1H, s), 7.03 (2H, s), 7.63 (2H, d, J=9Hz), 8.20 (2H, d, J=9Hz)
Mass : M⁺¹ 268, M 267, m/e 237, 177, 113

### Example 7

To a mixture of 2-amino-4-(4-nitrophenylthiomethyl)-thiazole (15 g) and ammonium chloride (2 g) in a mixture of tetrahydrofuran (100 ml), ethanol (150 ml) and water was added portionwise added the iron powder (15 g) at 80°C with stirring. The mixture was refluxed for 2 hours with stirring. The reaction mixture was filtered by suction and the filtrate was concentrated under reduced pressure. The residue was extracted with a mixture of tetrahydrofuran and ethyl acetate (1:1), washed with saturated aqueous sodium chloride and dried over magnesium sulfate. The solvent was evaporated in vacuo and the residue was triturated with chloroform. The precipitates were collected by filtration, washed with ether and dried in vacuo to give 2-amino-4-(4-aminophenylthiomethyl)-thiazole (10.50 g, yield : 73.0%).
mp : 130-132°C
IR (Nujol) : 3425, 3350, 1630, 1605, 1595, 1535, 1495, 1440, 1380, 1340, 1280 cm⁻¹
NMR (DMSO-d₆, 90MHZ, ppm) : 3.70 (2H, s), 5.15 (2H, s), 6.10 (1H, s), 6.45 (2H, d, J=9Hz), 6.83 (2H, s), 7.00 (2H, d, J=9Hz)
Mass : M⁺¹ 238, M 237, m/e 204, 124, 113

### Example 8

A solution of 3-chloroperbenzoic acid (4.9 g) in dichloromethane (100 ml) was dropwise added to a solution of 2-amino-4-(4-aminophenylthiomethyl)thiazole (5.1 g) in a mixture of dichloromethane (200 ml) and N,N-dimethylformamide (10 ml) at 5°C with stirring. The mixture was stirred at 5°C for 1.5 hours with stirring. The precipitates were collected by filtration, washed with ethyl acetate and dried in vacuo to give solid. The solid was recrystallized from ethanol to give 2-amino-4-(4-aminophenylsulfinylmethyl)thiazole (4.70 g, yield : 86.3%).
IR (Nujol) : 3350-3100, 1620, 1600, 1500, 1380, 1300 cm⁻¹
NMR (DMSO-d₆, 60MHZ, ppm) : 3.87 (2H, s), 6.27 (2H, s), 6.67 (2H, d, J=9Hz), 7.00 (2H, s), 7.30 (2H, d, J=9Hz), 7.67 (1H, s)
Mass : M⁺ 254, M 253, m/e 237, 205, 156, 139

### Example 9

To a solution of 2-amino-4-(4-aminophenylsulfinylmethyl)thiazole (2.8 g) in N,N-dimethylformamide (30 ml) was added portionwise the 3-chloroperbenzoic acid (2.6 g) at 5°C with stirring. The mixture was stirred at room temperature for 2 hours and then the solution was poured into ice-water. The precipitates were collected by filtration, washed with aqueous sodium bicarbonate, washed with water and dried in vacuo to give 2-amino-4-(4-aminophenylsulfonylmethyl)-thiazole (2.85 g, yield 95.6%).
mp : 204-208°C (dec.)
IR (Nujol) : 3375, 3275, 3150, 1615, 1595, 1295, 1140 cm⁻¹
NMR (DMSO-d₆, 60MHZ, ppm) : 4.30 (2H, s), 6.10 (2H, s), 6.30 (1H, s), 6.67 (2H, d, J=8Hz), 6.95 (2H, s), 7.43 (2H, d, J=8Hz)
Mass : M 269, m/e 220, 205

### Example 10

A mixture of 2-acetylamino-5-chlorothiazole (5.3 g), 4-mercaptopyridine (3.4 g) and potassium carbonate in N,N-dimethylformamide (50 ml) was heated at 120°C for 2.5 hours with stirring. The reaction mixture was concentrated under reduced pressure and the residue was triturated with water. The precipitates were collected by filtration, washed with water and dried in vacuo to give 2-acetylamino-5-(4-pyridylthio)thiazole (6.3 g, yield : 83.7%).
IR (Nujol) : 3150, 1680, 1580, 1300 cm⁻¹
NMR (DMSO-d₆, 90MHZ, ppm) : 2.23 (3H, s), 7.10 (2H, d, J=6Hz), 7.80 (1H, s), 8.40 (2H, d, J=6Hz), 11.90 (1H, br s)

### Example 11

A mixture of 2-acetylamino-5-(4-pyridylthio)thiazole (4.7 g), acetic acid (35 ml) and 6N-aqueous hydrochloric acid (10 ml) was refluxed for 2 hours with stirring. The reaction mixture was concentrated under reduced pressure and the residue was dissolved in water. The solution was adjusted pH 8.5 using aqueous sodium bicarbonate under ice cooling. The precipitates were collected by filtration, washed with water and dried in vacuo to give 2-amino-5-(4-pyridylthio)thiazole (2.7 g, yield : 69.5%).
mp : 180-185°C (dec.)
IR (Nujol) : 3270, 3150, 1630, 1580, 1380 cm⁻¹
NMR (DMSO-d₆, 90MHZ, ppm) : 7.13 (2H, d, J=6Hz), 7.30 (1H, s), 7.60 (2H, s), 8.40 (2H, d, J=6Hz)
Mass : M⁺¹ 210, M 209, m/e 188, 150, 131, 99

### Example 12

To a mixture of 2-amino-5-(4-pyridylthio)thiazole (4.0 g) in a mixture of chloroform (300 ml) and N,N-dimethylformamide (10 ml) was added dropwise the solution of 3-chloroperbenzoic acid (4.5 g) in chloroform (100 ml) at 5°C with stirring. The mixture was stirred at 5°C for 26 hours under ice cooling. The reaction mixture was washed with aqueous sodium bicarbonate and water and dried over magnesium sulfate. The solvent was concentrated under reduced pressure to give solid. The solid was subject to column chromatography on silica gel (silica gel 60, 70-230 mesh; Merck : 250 g) and eluted with a mixture of chloroform and methanol (10:1). The fractions containing the objective compound were combined and concentrated under reduced pressure to give 2-amino-5-(4-pyridylsulfinyl)thiazole (2.5 g, yield: 58.1%).
mp : 193-195°C
IR (Nujol) : 3350, 3250, 1610, 1575, 1525, 1280, 1220 cm⁻¹
NMR (DMSO-d₆, 60MHZ, ppm) : 7.62 (2H, d, J=6Hz), 7.87 (1H, s), 7.97 (2H, s), 8.80 (2H, d, J=6Hz)
Mass : M 225, m/e 209, 177, 147, 131

### Example 13

A mixture of 2-acetylamino-5-chlorothiazole (5.3 g), 2-mercaptopyridine (3.5 g) and potassium carbonate (6.2 g) in N,N-dimethylformamide (50 ml) was heated at 130°C for 3.5 hours with stirring. The reaction mixture was concentrated under reduced pressure and the residue was triturated with water. The precipitates were collected by filtration, washed with water and dried in vacuo to give 2-acetylamino-5-(2-pyridylthio)thiazole (5.70 g, yield : 76.0%).
mp : 185-188°C (dec.)
IR (Nujol) : 3150, 1695, 1575, 1300, 1280, 1230 cm⁻¹
NMR (DMSO-d₆, 60MHZ, ppm) : 2.20 (3H, s), 7.00-7.40 (2H, m), 7.70-7.90 (2H, m), 8.50 (1H, m), 12.40 (1H, s)
Mass : M⁺¹ 252, M 251, m/e 209, 176, 167

### Example 14

A mixture of 2-acetylamino-5-(2-pyridylthio)thiazole (5.0 g) in a mixture of acetic acid (50 ml) and aqueous 6N-hydrochloric acid (10 ml) was refluxed for 2 hours with stirring. The reaction mixture was concentrated under reduced pressure and the residue was dissolved in water. The solution was adjusted to pH 8.5 using aqueous sodium bicarbonate, and then the mixture was extracted with ethyl acetate. The organic layer was washed with water and dried over magnesium sulfate. The solvent was concentrated under reduced pressure and the residue was triturated with a solution of hydrochloric acid in ethanol. The precipitates were collected by filtration, washed with isopropyl ether and dried in vacuo to give 2-amino-5-(2-pyridylthio)thiazole dihydrochloride (4.60 g, yield : 85.8%).
mp : 220-225°C (dec.)
IR (Nujol ) : 2550-2300, 1620, 1595 cm⁻¹
NMR (DMSO-d₆, 60MHZ, ppm) : 7.20-7.60 (2H, m), 7.70-8.00 (4H, m), 8.55 (1H, m), 10.50 (3H, br s)
Mass : m/e 209 (free), 187, 167, 123

### Example 15

To a solution of 2-amino-5-(2-pyridylthio)thiazole dihydrochloride (4.0 g) in chloroform (100 ml) was dropwise added the solution of 3-chloroperbenzoic acid (5.0 g) in chloroform (100 ml) at 5°C with stirring. The mixture was stirred at 5°C for 1.5 hours. The reaction mixture was washed with aqueous sodium bicarbonate and dried over magnesium sulfate. The solvent was concentrated under reduced pressure to give solid. The solid was subjected to column chromatography on silica gel (silica gel 60, 70-230 mesh; Merck : 100 g) and eluted with a mixture of chloroform and methanol (10:1). The fractions containing the objective compound were combined and concentrated under reduced pressure to give 2-amino-5-(2-pyridylsulfinyl)thiazole (3.4 g, yield : 78.9%).
mp : 200-202°C (dec.)
IR (Nujol) : 3300, 3150, 1630, 1575, 1270, 1225 cm⁻¹
NMR (DMSO-d₆, 90MHZ, ppm) : 7.40-7.60 (1H, m), 7.70 (1H, s), 7.73 (2H, s), 7.90-8.20 (2H, m), 8.60 (1H, m)
Mass : M 225, m/e 209, 147, 115

### Example 16

To a solution of 2-amino-5-(2-pyridylthio)thiazole (5.7 g) in chloroform (450 ml) was dropwise added the solution of 3-chloroperbenzoic acid (15 g) in chloroform (100 ml) at 5°C with stirring. The mixture was stirred at room temperature for 16 hours. The reaction mixture was washed with aqueous sodium bicarbonate and dried over magnesium sulfate. The solvent was concentrated under reduced pressure to give 2-amino-5-(2-pyridylsulfonyl)-thiazole (2.2 g, yield : 33%).
mp : 178-182°C (dec.)
IR (Nujol) : 3375, 3300, 3150, 1645, 1610, 1525, 1320, 1220 cm⁻¹
NMR (DMSO-d₆, 60MHZ, ppm) : 7.55-7.80 (2H, m), 8.00-8.30 (4H, m), 8.77 (1H, m)
Mass : M⁺¹ 242, M 241, m/e 177, 156, 135

### Example 17

A mixture of 2-acetamido-5-chlorothiazole (14.3 g), 2-mercaptopyrimidine (10 g) and potassium carbonate anhydrous (22.4 g) in N,N-dimethylformamide (280 ml) was stirred at 150°C an hour. The reaction mixture was poured into water with stirring under ice cooling. The mixture was extracted with ethyl acetate, washed with water and dried over magnesium sulfate. The organic layer was concentrated under reduced pressure to give solid. The solid was triturated with water, and the precipitates were collected by filtration, washed with water and dried in vacuo to give 2-acetamido-5-(2-pyrimidinylthio)thiazole (12.30 g, yield : 60.2%).
mp : 225°C (dec.)
IR (Nujol) : 3170, 1645, 1555, 1310 cm⁻¹
NMR (DMSO-d₆, 60MHZ, ppm) : 2.20 (3H, s), 7.33 (lH, t, J=4Hz), 7.70 (1H, s), 8.68 (2H, d, J=4Hz), 12.33 (1H, br s)
Mass : M⁺ 254, M⁺¹ 253, M 252, m/e 210, 168

### Example 18

Starting from 2-acetamido-5-(2-pyrimidinylthio)thiazole, 2-amino-5-(2-pyrimidinylthio)thiazole (2.02 g, yield : 22.0%) was obtained
mp : 175-177°C
IR (Nujol) : 3270, 3100, 1655, 1565, 1555, 1540 cm⁻¹
NMR (DMSO-d₆, 60MHZ, ppm) : 7.13-7.57 (4H, m), 8.40-8.77 (2H, m)
Mass : M⁺ 212, M⁺¹ 211, M 210, m/e 168, 124

### Example 19

A mixture of 2-acetylamino-5-chlorothiazole (5.3 g), 1-methyl-2-mercaptoimidazole (3.6 g) and potassium carbonate (6.2 g) in N,N-dimethylformamide (50 ml) was heated at 130°C for 5.5 hours with stirring. The reaction mixture was concentrated under reduced pressure and the residue was triturated with water. The precipitates were collected by filtration washed with water and dried in vacuo to give 2-acetylamino-5-(1-methylimidazol-2-ylthio)-thiazole (6.95 g, yield : 91.2%).
mp : 155-160°C (dec.)
IR (Nujol) : 3400, 1690, 1565, 1300 cm⁻¹
NMR (DMSO-d₆, 90MHZ, ppm) : 2.10 (3H, s), 3.70 (3H, s), 6.90 (lH, s), 7.26 (1H, s), 7.60 (1H, s)
Mass : M⁺¹ 255, M 254, m/e 212, 179, 170, 114 was obtained by refluxing in a mixture of ethanol and concentrated hydrochloric acid with stirring. The reaction mixture was concentrated under reduced pressure and the residue was dissolved in water. The solution was adjusted to pH 12 using aqueous sodium hydroxide with stirring under ice-cooling. The mixture was extracted with ethyl acetate, washed with water and dried over magnesium sulfate. The organic layer was concentrated under reduced pressure to give solid. The solid was subjected to column chromotography on silica gel and eluted with a mixture of chloroform and methanol (100 : 1). The fractions containing the objective compounds were combined and evaporated to dryness in vacuo to give the desired compound.

### Example 20

A solution of 2-acetylamino-5-(1-methylimidazol-2-ylthio)thiazole (7.0 g) in a mixture of acetic acid (100 ml) and aqueous 6N-hydrochloric acid (20 ml) was refluxed for 3.5 hours with stirring. The reaction mixture was concentrated under reduced pressure and the residue was adjusted to pH 8 using aqueous sodium bicarbonate under ice cooling. The precipitates were collected by filtration, washed with water and dried in vacuo to give 2-amino-5-(l-methylimidazol-2-ylthio)thiazole (4.9 g, yield : 83.9%).
mp : 180-190°C (dec.)
IR (Nujol) : 3300, 3150, 1620, 1530, 1280, 1220 cm⁻¹
NMR (DMSO-d₆, 60MHZ, ppm) : 3.77 (3H, s), 7.03 (1H, s), 7.25 (1H, s), 7.37 (1H, s)
Mass : M⁺¹ 213, M 212, m/e 179, 170, 126, 114

### Example 21

To a mixture of 2-amino-5-(4-pyridylthio)thiazole (2.5 g) and pyridine (3 g) in N,N-dimethylformamide (25 ml) was added dropwise the 4-fluorobenzoyl chloride (2.7 g) at 5°C under ice cooling with stirring. The mixture was stirred at 5°C for 4 hours under ice cooling. The reaction mixture was concentrated under reduced pressure and the residue was triturated with water. The precipitates were collected by filtration, washed with water and dried in vacuo to give 2-(4-fluorobenzoylamino)-5-(4-pyridylthio)thiazole (2.5 g, yield : 63.1%).
mp : 220-225°C (dec.)
IR (Nujol) : 3150, 1670, 1605, 1587, 1550, 1295, 1230 cm⁻¹
NMR (DMSO-d₆, 60MHZ, ppm) : 7.10-7.67 (4H, m), 7.95 (1H, s), 8.10-8.60 (4H, m), 12.85 (1H, s)
Mass : M⁺¹ 332, M 331, m/e 209, 123, 95

### Example 22

A mixture of 4-chloromethyl-2-formylaminothiazole (1.86 g), 4-mercaptopyridine (1.23 g) and potassium carbonate (1.8 g) in N,N-dimethylformamide (20 ml) was heated at 100°C for 2 hours with stirring. The reaction mixture was concentrated under reduced pressure and the residue was triturated with water. The precipitates were collected by filtration, washed with water and dried in vacuo to give 2-formylamino-4-(4-pyridylthiomethyl)-thiazole (1.7 g, yield : 68.0%).
mp : 182-184°C
IR (Nujol) : 1675, 1650, 1585, 1560, 1270 cm⁻¹
NMR (DMSO-d₆, 60MHZ, ppm) : 4.30 (2H, s), 7.15 (1H, s), 7.33 (2H, d, J=6Hz), 8.33 (2H, d, J=6Hz), 8.45 (1H, s)
Mass : M⁺¹ 252, M 251, m/e 223, 155, 141, 113

### Example 23

A mixture of 2-formylamino-4-(4-pyridylthiomethyl)-thiazole (1.6 g) and N-chlorosuccinimide (1.5 g) in acetic acid (25 ml) was heated at 40-50°C for 5 hours with stirring and then the mixture was allowed to stand at room temperature for 16 hours. The reaction mixture was concentrated under reduced pressure and the residue was triturated with aqueous sodium bicarbonate. The precipitates were collected by filtration washed with water and dried in vacuo to give 5-chloro-2-formylamino-4-(4-pyridylthiomethyl)thiazole (0.85 g, yield : 46.8%).
mp : 200-203°C (dec.)
IR (Nujol) : 1680, 1665, 1587, 1300 cm⁻¹
NMR (DMSO-d₆, 60MHZ, ppm) : 4.37 (2H, s), 7.40 (2H, d, J=6Hz), 7.47 (2H, d, J=6Hz), 8.53 (1H, s), 12.50 (1H, s)
Mass : M⁺³ 288, M⁺ 287, M⁺¹ 286, M 285, m/e 256, 250, 175, 147

### Example 24

A solution of 5-chloro-2-formylamino-4-(4-pyridylthiomethyl)thiazole (4.9 g) in a mixture of ethanol (25 ml), tetrahydrofuran (20 ml) and concentrated hydrochloric acid (7 ml) was stirred at room temperature for 4 hours. The reaction mixture was concentrated under reduced pressure and the residue was dissolved in water. The solution was adjusted to pH 8 using aqueous sodium bicarbonate under ice-cooling. The precipitates were collected by filtration, washed with water and dried in vacuo to give 2-amino-5-chloro-4-(4-pyridylthiomethyl)-thiazole (0.26 g, yield : 56.6%).
IR (Nujol) : 3350, 3250, 1685, 1530 cm⁻¹
NMR (DMSO-d₆, 60MHZ, ppm) : 4.13 (2H, s), 7.33 (2H, d, J=6Hz), 7.43 (2H, s), 8.50 (2H, d, J=6Hz)
Mass : M⁺ 259, M⁺¹ 258, M 257, m/e 220, 147, 111

### Example 25

A mixture of 4-chloromethyl-2-formylaminothiazole (1.76 g), 4-nitrothiophenol (1.7 g) and potassium carbonate (1.8 g) in N,N-dimethylformamide (20 ml) was heated at 100°C with stirring. The reaction mixture was poured into ice-water and stirred at 5°C for an hour. The precipitates were collected by filtration, washed with water and dried in vacuo to give 2-formylamino-4-(4-nitrophenylthiomethyl)thiazole (2.3 g, yield : 78%).
mp : 158-160°C
IR (Nujol) : 3500, 1680, 1595, 1550, 1330 cm⁻¹
NMR (DMSO-d₆, 60MHZ, ppm) : 4.40 (2H, s), 7.13 (1H, s), 7.56 (2H, d, J=8Hz), 8.10 (2H; d, J=8Hz), 8.50 (1H, s)
Mass : M 295, m/e 265, 141, 113

### Example 26

To a mixture of 2-formylamino-4-(4-nitrophenylthiomethyl)thiazole (2.2 g) and ammonium chloride (0.5 g) in a mixture of tetrahydrofuran (30 ml), ethanol (50 ml) and water (10 ml) was added portionwise the iron powder at 80°C with stirring. The mixture was refluxed for 2 hours with stirring. The reaction mixture was filtered by suction and the residue was triturated with water. The precipitates were collected by filtration, washed with water and dried in vacuo to give 2-formylamino-4-(4-aminophenylthiomethyl)thiazole (1.6 g, yield : 81%).
mp : 180-182°C
IR (Nujol) : 3350, 3300, 1680, 1625, 1600, 1325, 1290 cm⁻¹
NMR (DMSO-d₆, 60MHZ, ppm) : 4.00 (2H, s), 5.23 (2H, s), 6.57 (2H, d, J=8Hz), 6.83 (1H, s), 7.10 (2H, d, J=8Hz), 8.50 (1H, s)
Mass : M⁺¹ 266, M 265, m/e 237, 205, 141, 124

### Example 27

A mixture of acetic anhydride (1.84 g) and formic acid (0.9 g) was heated at 50°C for 0.5 hours with stirring. The solution was cooled at room temperature and to the solution was added the 2-formylamino-4-(4-aminophenylthiomethyl)thiazole (1.6 g). The mixture was stirred at room temperature for 6.5 hours and then the mixture was poured into ice-water. The precipitates were collected by filtration, washed with water and dried in vacuo to give 2-formylamino-4-(4-formylaminophenylthiomethyl)thiazole (1.7 g, yield : 96.7%).
mp : 195-197°C (dec.)
IR (Nujol) : 3150, 1680, 1660, 1595, 1525, 1310, 1290 cm⁻¹
NMR (DMSO-d₆, 60MHZ, ppm) : 4.30 (2H, s), 7.10 (1H, s), 7.47 (2H, d, J=8Hz), 7.73 (2H, d, J=8Hz), 8.40 (1H, s), 8.60 (1H, s), 10.33 (1H, s), 12.20 (1H, s)
Mass : M⁺¹ 294, M 293, m/e 265, 153, 141, 113

### Example 28

To a solution of 2-formylamino-4-(4-formylaminophenylthiomethyl)thiazole (2.9 g) in acetic acid (30 ml) was portionwise added N-chlorosuccinimide at 50°C with stirring. The mixture was heated at 50°C with stirring. The reaction mixture was concentrated under reduced pressure and the residue was triturated with water. The mixture was extracted with a mixture of ethyl acetate and tetrahydrofuran (1:1), washed with water and dried over magnesium sulfate. The solvent was concentrated under reduced pressure to give solid. The solid was subjected to column chromatography on silica gel (silica gel 60, 70-230 mesh; Merck : 150 g) and eluted with a mixture of chloroform and methanol (10:1). The fractions containing the objective compound were combined and concentrated under reduced pressure to give 5-chloro-2-formylamino-4-(4-formylaminophenylthiomethyl)thiazole (2.0 g, yield : 61.2%).
mp : 130-150°C (dec.)
IR (Nujol) : 3350, 3200, 1710, 1690-1640, 1590 cm⁻¹
NMR (DMSO-d₆, 90MHZ, ppm) : 4.80 (2H, s), 7.26 (2H, d, J=8Hz), 7.56 (2H, d, J=8Hz), 8.25 (1H, s), 8.50 (1H, s), 10.23 (1H, s), 12.57 (1H, s)
Mass : M 327, m/e 298, 292, 263, 234, 204

### Example 29

A solution of 5-chloro-2-formylamino-4-(4-formylaminophenylthiomethyl)thiazole (3.5 g) in a mixture of concentrated hydrochloric acid (9 ml), methanol (30 ml) and tetrahydrofuran (30 ml) was stirred at room temperature for 4 hours. The reaction mixture was concentrated under reduced pressure and the residue was dissolved in water. The solution was adjusted to pH 8 using aqueous sodium bicarbonate with stirring under ice cooling. The precipitates were collected by filtration, washed with water and dried in vacuo to give solid. The solid was subjected to column chromatography on silica gel (silica gel 60, 70-230 mesh; Merck : 150 g) and eluted with a mixture of chloroform and methanol (10:1). The fractions containing the objective compound were combined and concentrated under reduced pressure to give 2-amino-4-(4-aminophenylthiomethyl)-5-chlorothiazole (2.3 g, yield : 79.3%).
mp : 158-163°C (dec.)
IR (Nujol) : 3325, 3200, 3150, 1620, 1595, 1495, 1325, 1290 cm⁻¹
NMR (DMSO-d₆, 60MHZ, ppm) : 4.33 (2H, s), 5.50 (2H, br s), 6.60 (2H, d, J=8Hz), 7.10 (2H, d, J=8Hz), 7.25 (2H, br s)
Mass : M 271, m/e 267, 236, 221, 204, 124

### Example 30

A mixture of 2-acetylamino-5-chloro-4-hydroxymethylthiazole (1 g), 4-mercaptopyridine (0.6 g) and potassium carbonate (1 g) in N,N-dimethylformamide (20 ml) was heated at 110°C for 8 hours with stirring. The reaction mixture was poured into ice water and filtered by suction. The filtrate was extracted with a mixture of ethyl acetate and tetrahydrofuran (1:1) and dried over magnesium sulfate. The solvent was concentrated under reduced pressure to give solid. The solid was subjected to column chromatography on silica gel (silica gel 60, 70-230 mesh; Merck : 75 g) and eluted with a mixture of chloroform and methanol (10:1). The fractions containing the objective compound were combined and concentrated under reduced pressure to give 2-acetylamino-4-hydroxymethyl-5-(4-pyridylthio)thiazole (0.90 g, yield : 64.3%).
mp : 220-222°C (dec.)
NMR (DMSO-d₆, 90MHZ, ppm) 2.16 (3H, s), 4.40 (2H, d, J=6Hz), 5.13 (1H, t, J=6Hz), 7.05 (2H, d, J=6Hz), 8.30 (2H, d, J=6Hz), 12.43 (1H, s)
Mass : M⁺¹ 282, M 281, m/e 239, 220, 205, 188

### Example 31

A mixture of 2-acetylamino-4-hydroxymethyl-5-(4-pyridylthio)thiazole (3.0 g) in a mixture of concentrated hydrochloric acid (8 ml) and ethanol (100 ml) was refluxed for 2.5 hours with stirring. The reaction mixture was concentrated under reduced pressure and the residue was triturated with acetone. The precipitates were collected by filtration, washed with isopropyl ether and were recrystallized from a mixture of ethanol and isopropyl ether to give 2-amino-4-hydroxymethyl-5-(4-pyridylthio)thiazole dihydrochloride (2.5 g, yield : 75.3%).
mp: 231-237°C (dec.)
IR (Nujol) : 3350, 2300, 1610, 1560 cm⁻¹
NMR (DMSO-d₆, 90MHZ, ppm) : 4.30-4.55 (3H, m), 7.80 (2H, d, J=6Hz), 8.65 (2H, d, J=6Hz), 8.83 (4H, br s)
Mass : M 239, m/e 222, 210, 188

### Example 32

Starting from 2-amino-5-(4-pyridylthio)thiazole 2-amino-5-(4-pyridylsulfonyl)thiazole (0.73 g, yield : 17.1%) was obtained according to a similar manner to that of Example 16.
mp : 217°C (dec.)
IR (Nujol) : 3260, 3100, 1620, 1580, 1525 cm⁻¹
NMR (DMSO-d₆, 90MHZ, ppm) : 7.73-7.86 (3H, m), 8.20 (2H, br s), 8.55 (2H, d, J=6Hz)
Mass : M⁺ 243, M⁺¹ 242, M⁺ 241, m/e 209, 195

### Example 33

A mixture of 2-acetylamino-5-chlorothiazole (1.76 g), 5-mercapto-2-methyl-1,3,4-thiadiazole (1.3 g) and potassium carbonate (2.0 g) in N,N-dimethylformamide (40 ml) was heated at 120°C for 4 hours with stirring. The reaction mixture was concentrated under reduced pressure and water was added to this residue. The mixture was extracted with a mixture of tetrahydrofuran and ethyl acetate (1:1), washed with aqueous saturated sodium chloride and dried over magnesium sulfate. The solvent was concentrated under reduced pressure to give solid. The solid was subjected to column chromatography on silica gel (silica gel 60, 70-230 mesh; Merck : 150 g) and eluted with a mixture of chloroform and methanol (10:1). The fractions containing the objective compound were combined and concentrated under reduced pressure to give 2-acetylamino-5-(2-methyl-1,3,4-thiadiazol-5-ylthio)-thiazole (1.65 g, yield : 60.7%).
mp : 242-244°C
IR (Nujol) : 3250, 1695, 1550, 1300 cm⁻¹
NMR (DMSO-d₆, 200MHZ, ppm) : 2.19 (3H, s), 2.63 (3H, s), 7.95 (1H, s), 12.58 (1H, s)
Mass : M⁺¹ 273, M 272, m/e 230, 188, 155, 131

### Example 34

A mixture of 2-acetylamino-5-(2-methyl-1,3,4-thiadiazol-5-ylthio)thiazole (3.3 g) in a mixture of ethanol (70 ml), tetrahydrofuran (50 ml) and aqueous 6N-hydrochloric acid (200 ml) was refluxed for 6.5 hours with stirring. The reaction mixture was concentrated under reduced pressure and the residue was dissolved in water. The solution was adjusted to pH 8.5 using aqueous sodium bicarbonate and extracted with a mixture of tetrahydrofuran and ethyl acetate (1:1). The organic layer was washed with aqueous saturated sodium chloride and dried over magnesium sulfate. The organic solvent was concentrated under reduced pressure to give solid. The solid was subjected to column chromatography on silica gel (silica gel 60, 70-230 mesh; Merck : 150 g) and eluted with a mixture of chloroform and methanol (10:1). The fractions containing the objective compound were combined and concentrated under reduced pressure to give 2-amino-5-(2-methyl-1,3,4-thiadiazol-5-ylthio)thiazole (0.85 g, Yield : 58.2%).
mp : 203-205°C (dec.)
IR (Nujol) : 3450, 3300, 3100, 1640, 1520, 1485, 1220 cm⁻¹
NMR (DMSO-d₆, 200MHZ, ppm) : 2.63 (3H, s), 7.42 (1H, s), 7.75 (2H, s)
Mass : M⁺¹ 231, M 230, m/e 188, 154, 131, 113

### Example 35

A mixture of 2-acetylamino-5-chlorothiazole (1.76 g), 5-mercapto-1-methyl-1H-tetrazole (1.2 g) and potassium carbonate (2 g) in N,N-dimethylformamide (40 ml) was heated at 130°C for 3 hours with stirring. The reaction mixture was concentrated under reduced pressure and the residue was triturated with water. The precipitates were collected by filtration, washed with water and dried in vacuo to give solid. The solid was subjected to column chromatography on silica gel (silica gel 60, 70-230 mesh; Merck : 150g) and eluted with a mixture of chloroform and methanol (10:1). The fractions containing the objective compound were combined and concentrated under reduced pressure to give 2-acetylamino-5-(1-methyl-1H-tetrazol-5-ylthio)thiazole (2.1 g, yield : 82.0%).
mp : 208-210°C
IR (Nujol) : 3450, 3250, 3150, 1690, 1665, 1550, 1295, 1225 cm⁻¹
NMR (DMSO-d₆, 200MHZ, ppm) : 2.17 (3H, s), 4.11 (3H, s), 7.89 (1H, s), 12.51 (1H, s)
Mass : M⁺¹ 257, M 256, m/e 214, 173, 159, 131

### Example 36

A mixture of 2-acetylamino-5-(1-methyl-1H-tetrazol-5-ylthio)thiazole (2.0 g) in a mixture of ethanol (20 ml) and aqueous 6N-hydrochloric acid (5 ml) was refluxed for 4 hours with stirring. The reaction mixture was concentrated under reduced pressure and the residue was adjusted to pH 8 using aqueous sodium bicarbonate under ice cooling. The precipitates were collected by filtration, washed with water and the solid was recrystallized from ethanol to give 2-amino-5-(1-methyl-lH-tetrazol-5-ylthio)thiazole (0.81 g, yield : 48.5%).
mp : 186-188°C (dec.)
IR (Nujol) : 3400, 3250, 3150, 1612, 1510, 1490, 1215 cm⁻¹
NMR (DMSO-d₆, 200MHZ, ppm) : 4.03 (3H, s), 7.38 (1H, s), 7.63 (2H, s)
Mass : M⁺¹ 215, M 214, m/e 131, 89, 83

### Example 37

A mixture of 2-amino-5-bromothiazole hydrochloride (2.2 g), 4-amino-2-mercaptopyrimidine (2.2 g) and potassium carbonate (6.5 g) in N,N-dimethylformamide (100 ml) was heated at 90°C for 2.5 hours with stirring. The reaction mixture was concentrated under reduced pressure and water was added to this residue. The solution was extracted with a mixture of tetrahydrofuran and ethyl acetate (1:1), washed with aqueous saturated sodium chloride and dried over magnesium sulfate. The solvent was concentrated under reduced pressure to give solid. The solid was subjected to column chromatography on silica gel (silica gel 60, 70-230 mesh; Merck : 250 g) and eluted with a mixture of chloroform and methanol (10:1). The fractions containing the objective compound were combined and concentrated under reduced pressure to give solid. The solid was triturated with ethanol to give 2-amino-5-(4-aminopyrimidin-2-ylthio)thiazole (1.25 g, yield : 55.6%).
mp : 185-187°C (dec.)
IR (Nujol) : 3450, 3300, 3175, 3100, 1645, 1630, 1580, 1545, 1340 cm⁻¹
NMR (DMSO-d₆, 200MHZ, ppm) : 6.16 (1H, d, J=6Hz), 6.99 (2H, s), 7.07 (1H, s), 7.32 (2H, s), 7.86 (1H, d, J=6Hz)
Mass : M⁺¹ 226, M 225, m/e 183, 139

### Example 38

A mixture of 2-amino-5-bromo-4-methylthiazole hydrochloride (1.15 g), 2-mercaptopyrimidine (0.6 g) and potassium carbonate (1.7 g) in N,N-dimethylformamide (20 ml) was heated <t 90°C for 3.5 hours with stirring. The reaction mixture was poured into ice water. The mixture was extracted with a mixture of tetrahydrofuran and ethyl acetate (1:1), washed with aqueous saturated sodium chloride and dried over magnesium sulfate. The solvent was concentrated under reduced pressure to give solid. The solid was subjected to column chromatography on silica gel (silica gel 60, 70-230 mesh; Merck : 100 g) and eluted with a mixture of chloroform and methanol (10:1). The fractions containing the objective compound were combined and concentrated under reduced pressure to give 2-amino-4-methyl-5-(2-pyrimidinylthio)thiazole (0.65 g, yield : 58.0%).
mp : 165-170°C (dec.)
IR (Nujol) : 3300, 3175, 1630, 1555, 1490, 1320 cm⁻¹
NMR (DMSO-d₆, 200MHZ, ppm) : 2.10 (3H, s), 7.24-7.33 (1H, m), 7.33 (2H, s), 8.64 (2H, d, J=5Hz)
Mass : M⁺¹ 225, M 224, m/e 209, 191, 182, 166, 145

### Example 39

A mixture of 2-amino-5-bromo-4-methylthiazole hydrochloride (4.5 g), 2-mercaptopyridine (2.3 g) and potassium carbonate (7.0 g) in N,N-dimethylformamide (100 ml) was heated at 90°C for 3 hours with stirring. The reaction mixture was concentrated under reduced pressure and water was added to this residue. The mixture was extracted with a mixture of tetrahydrofuran and ethyl acetate, washed with aqueous saturated sodium chloride and dried over magnesium sulfate. The solvent was concentrated under reduced pressure to give solid. The solid was subjected to column chromatography on silica gel (silica gel 60, 70-230 mesh; Merck : 300 g) and eluted with a mixture of chloroform and methanol (10:1). The fractions containing the objective compound were combined and concentrated under reduced pressure to give oil. Again the oil was subjected to column chromatography on silica gel (silica gel 60, 70-230 mesh; Merck : 200 g) and eluted with a mixture of dichloromethane and acetone (5:1). The fractions containing the objective compound were combined and concentrated under reduced pressure to give 2-amino-4-methyl-5-(2-pyridylthio)thiazole (2.1 g, yield : 47.9%).
NMR (DMSO-d₆, 200MHZ, ppm) : 2.13 (3H, s), 6.97 (1H, m), 7.15 (1H, m), 7.28 (2H, s), 7.65 (1H, m), 8.40 (1H, m)
Mass : M⁺¹ 224, M 223, m/e 208, 190, 181, 145, 111

### Example 40

A mixture of 2-amino-4-methyl-5-(2-pyridylthio)-thiazole (1.7 g) and 3-chloroperbenzoic acid (1.8 g) in a mixture of chloroform (20 ml) and dichloromethane (50 ml) was stirred at 5°C for 3.5 hours. The reaction mixture was washed with aqueous sodium bicarbonate and dried over magnesium sulfate. The solvent was concentrated under reduced pressure to give solid. The solid was subjected to column chromatography on silica gel (silica gel 60, 70-230 mesh; Merck : 100 g) and eluted with a mixture of chloroform and methanol (10:1). The fractions containing the objective compound were combined and concentrated under reduced pressure to give 2-amino-4-methyl-5-(2-pyridylsulfinyl)thiazole (0.95 g, yield : 52.2 %).
mp : 190-193°C (dec.)
NMR (DMSO-d₆, 200MHz, ppm) : 2.38 (3H, s), 7.50-7.58 (1H, m), 7.70 (2H, s), 7.96 (1H, d, J=8Hz), 8.07-8.16 (1H, m), 8.6-8.63 (1H, m)
Mass : M⁺¹ 240, M 239, m/e 223, 191, 161, 129, 111

### Example 41

A mixture of 2-acetylamino-5-bromothiazole (1.9 g), 2-mercaptoimidazole (0.9 g) and potassium carbonate (1.5 g) in N,N-dimethylformamide (30 ml) was heated at 90°C for 2.5 hours with stirring. The reaction mixture was concentrated under reduced pressure and the residue was extracted with methanol. The solvent was concentrated under reduced pressure to give solid. The solid was subjected to column chromatography on silica gel (silica gel 60, 70-230 mesh; Merck : 150 g) and eluted with a mixture of chloroform and methanol (10:1). The fractions containing the objective compound were combined an concentrated under reduced pressure to give 2-acetylamino-5-(2-imidazolylthio)thiazole (1.8 g, yield : 87.4%).
mp : 230-235°C (dec.)
IR (Nujol) : 3150, 3100, 1710, 1550, 1290 cm⁻¹
NMR (DMSO-d₆, 200MHZ, ppm) : 2.1 (3H, s), 6.80 (1H, s), 7.08 (2H, s), 7.6 (1H, s), 12.3 (1H, s)
Mass : M⁺¹ 241, M 240, m/e 198, 156, 100

### Example 42

A mixture of 2-acetylamino-5-(2-imidazolylthio)-thiazole (1.8 g) in a mixture of concentrated hydrochloric acid (10 ml) and ethanol (50 ml) was refluxed for 5 hours with stirring. The reaction mixture was concentrated under reduced pressure and the residue was dissolved in water. The solution was adjusted to pH 8.5 using sodium bicarbonate with cooling. The precipitates were collected by filtration, washed with water and dried in vacuo to give 2-amino-5-(2-imidazolylthio)thiazole (0.35 g). The filtrate was extracted with a mixture of tetrahydrofuran and ethyl acetate (1:1), washed with aqueous saturated sodium chloride and dried over magnesium sulfate. The solvent was concentrated under reduced pressure to give solid. The solid was subjected to column chromatography on silica gel (silica gel 60, 70-230 mesh; Merck : 100 g) and eluted with a mixture of chloroform and methanol (10:1). The fractions containing the objective compound were combined and concentrated under reduced pressure to give 2-amino-5-(2-imidazolylthio)thiazole (0.55 g). Total amount of 2-amino-5-(2-imidazolylthio)thiazole was 0.90 g (yield : 60.4%).
mp : 209-211°C (dec.)
IR (Nujol) : 3450, 3300, 1630, 1520, 1315 cm⁻¹
NMR (DMSO-d₆, 200MHZ, ppm) : 7.05 (2H, s), 7.15 (1H, s), 7.36 (2H, s)
Mass : M⁺¹ 199, M 198, m/e 156, 139, 100

### Example 43

A mixture of 2-acetylamino-5-bromothiazole (1.8 g), 3-hydroxy-2-mercaptopyridine (1.3 g) and potassium carbonate (2.0 g) in N,N-dimethylformamide (40 ml) was heated at 90°C for 3.5 hours with stirring. The reaction mixture was concentrated under reduced pressure and the residue was triturated with water. The mixture was extracted with a mixture of tetrahydrofuran and ethyl acetate (1:1), washed with aqueous saturated sodium chloride and dried over magnesium sulfate. The solvent was concentrated under reduced pressure to give solid. The solid was subjected to column chromatography on silica gel (silica gel 60, 70-230 mesh; Merck : 150 g) and eluted with a mixture of chloroform and methanol (10:1). The fractions containing the objective compound were combined and concentrated under reduced pressure to give 2-acetylamino-5-(3-hydroxypyridin-2-ylthio)thiazole (2.4 g, yield : 89.9%).
mp : 236-238°C (dec.)
IR (Nujol) : 3175, 1690, 1565, 1300 cm⁻¹
NMR (DMSO-d₆, 200MHZ, ppm) : 2.16 (3H, s), 7.02-7.17 (3H, m), 7.58 (1H, s), 7.83 (1H, d, J=6Hz), 10.70 (1H, s), 12.30 (1H, s)
Mass : M⁺¹ 268, M 267, m/e 225, 183, 127

### Example 44

A mixture of 2-acetylamino-5-(3-hydroxypyridin-2-ylthio)thiazole (2 g) in a mixture of ethanol (40 ml), tetrahydrofuran (20 ml) and aqueous 6N-hydrochloric acid (13 ml) was refluxed for 5 hours with stirring. The reaction mixture was concentrated under reduced pressure and the residue was dissolved in water. The solution was adjusted to pH 8.5 using aqueous sodium bicarbonate and extracted with a mixture of tetrahydrofuran and ethyl acetate (1:1). The organic layer was washed with aqueous saturated sodium chloride and dried over magnesium sulfate. The solvent was concentrated under reduced pressure to give 2-amino-5-(3-hydroxypyridin-2-ylthio)-thiazole (1.15 g, yield : 68.9%).
mp : 128-130°C
IR (Nujol) : 3500, 3400, 3300, 1640, 1570, 1520, 1500, 1330, 1200 cm⁻¹
NMR (DMSO-d₆, 200MHZ, ppm) : 6.97-7.11 (3H, m), 7.29 (2H, s), 7.82 (1H, d, J=6Hz), 10.57 (1H, s)
Mass : M⁺¹ 226, M 225, m/e 183, 139, 100

### Example 45

To a mixture of 2-amino-5-(3-hydroxypyridin-2-ylthio)thiazole (4.6 g) in a mixture of chloroform (100 ml), dichloromethane (200 ml) and N,N-dimethylformamide (50 ml) was added dropwise the solution of 3-chloroperbenzoic acid (4.3 g) in chloroform (50 ml) at 5°C with stirring. The mixture was stirred at room temperature for 5 hours. The reaction mixture was extracted with aqueous diluted hydrochloric acid and the aqueous layer was washed with ethyl acetate. The aqueous layer was adjusted to pH 5.7 using sodium bicarbonate and extracted with a mixture of tetrahydrofuran and ethyl acetate (1:1). The organic layer was washed with aqueous saturated sodium chloride and dried over magnesium sulfate. The solvent was concentrated under reduced pressure to give solid. The solid was subjected to column chromatography on silica gel (silica gel 60, 70-230 mesh; Merck : 250 g) and eluted with a mixture of chloroform and methanol (10:1). The fractions containing the objective compound were combined and concentrated under reduced pressure to give 2-amino-5-(3-hydroxypyridin-2-ylsulfinyl)thiazole.(0.65 g, yield : 12.2%).
mp : 155-158°C (dec.)
IR (Nujol) : 3300, 3150, 1620, 1565, 1515, 1300 cm⁻¹
NMR (DMSO-d₆, 200MHZ, ppm) : 7.29-7.40 (2H, m), 7.59 (1H, s), 7.75 (2H, s), 8.15 (1H, br s)
Mass : m/e 225, 220, 205

### Example 46

A mixture of 2-acetylamino-5-bromothiazole (1 g), 3-mercaptopyridine hydrochloride (1 g) and potassium carbonate (1.5 g) in N,N-dimethylformamide (10 ml) was heated at 90°C for 4.5 hours with stirring. The reaction mixture was poured into ice water. The precipitates were collected by filtration, washed with water and dried in vacuo to give 2-acetylamino-5-(3-pyridylthio)thiazole (0.9 g, yield : 81.8%).
mp : 203-205°C (dec.)
IR (Nujol) : 3170, 1700, 1570, 1300 cm⁻¹
NMR (DMSO-d₆, 200MHZ, ppm) : 2.16 (3H, s), 7.33-7.39 (1H, s), 7.60 (1H, d, J=8Hz), 7.81 (1H, s), 8.42-8.54 (2H, m), 12.45 (1H, s)
Mass : M⁺¹ 252, M 251, m/e 209, 176, 167, 111

### Example 47

A mixture of 2-acetylamino-5-(3-pyridylthio)thiazole (8.5 g) in a mixture of ethanol (160 ml), tetrahydrofuran (50 ml) and aqueous 6N hydrochloric acid (100 ml) was refluxed for 4 hours with stirring. The reaction mixture was concentrated under reduced pressure and the residue was dissolved in water. The solution was adjusted to pH 8.5 using aqueous sodium bicarbonate and the precipitates were collected by filtration, washed with water and dried in vacuo to give 2-amino-5-(3-pyridylthio)thiazole (5.6 g yield : 78.9%).
mp : 140-142°C
IR (Nujol) : 3400, 3300, 3125, 1630, 1530, 1490 cm⁻¹
NMR (DMSO-d₆, 200MHZ, ppm) : 7.30 (1H, s), 7.32-7.39 (1H, m), 7.55 (2H, s), 7.57-7.61 (1H, m), 8.40 (2H, d, J=7Hz)
Mass : M⁺¹ 210, M 209, m/e 167, 122, 99

### Example 48

To a mixture of 2-amino-5-(3-pyridylthio)thiazole (3.0 g) in a mixture of dichloromethane (100 ml) and chloroform (100 ml) was added dropwise the solution of 3-chloroperbenzoic acid (3.4 g) in dichloromethane (50 ml) at 5°C with stirring. The mixture was stirred at 5°C for 3 hours. The reaction mixture was washed with aqueous sodium bicarbonate and the aqueous layer was extracted with a mixture of tetrahydrofuran and ethyl acetate (1:1), washed with aqueous saturated sodium chloride and dried over magnesium sulfate. The solvent was concentrated under reduced pressure and the residue was recrystallized from ethanol to give 2-amino-5-(3-pyridylsulfinyl)thiazole (1.2 g, yield : 37.2%).
mp : 178-179°C
IR (Nujol) : 3300, 3150, 1630, 1580, 1520, 1485, 1325, 1220 cm⁻¹
NMR (DMSO-d₆, 200MHZ, ppm) : 7.58-7.70 (1H, m), 7.80 (1H, s), 8.00 (2H, s), 7.98-8.05 (1H, m), 8.72 (2H, br s)
Mass : M⁺¹ 226, M 225, m/e 209, 177, 147

### Example 49

A mixture of 2-amino-5-(3-pyridylsulfinyl)thiazole (1.6 g) and 3-chloroperbenzoic acid (1.8 g) in a mixture of chloroform (150 ml), dichloromethane (50 ml) and N,N-dimethylformamide (5 ml) was stirred at room temperature for 3 hours. The reaction mixture was extracted with diluted hydrochloric acid and washed with ethyl acetate. The aqueous layer was adjusted to pH 8.5 using sodium bicarbonate and the mixture was extracted with a mixture of tetrahydrofuran and ethyl acetate (1:1). The organic layer was washed with water and dried over magnesium sulfate. The solvent was concentrated under reduced pressure and the residue was triturated with ethanol to give 2-amino-5-(3-pyridylsulfonyl)thiazole (0.30 g, yield : 17.5%).
mp : 218-220°C (dec.)
IR (Nujol) : 3420, 3300, 1650, 1520, 1310 cm⁻¹
NMR (DMSO-d₆, 200MHz, ppm) : 7.63-7.70 (1H, m), 7.78 (1H, s), 8.20 (2H, s), 8.27 (1H, d, J=8Hz), 8.85 (lH, d, J=4Hz), 9.06 (1H, s)
Mass : M⁺¹ 242, M 241, m/e 177, 135, 99

### Example 50

A mixture of 2-acetylamino-5-bromothiazole (2.2 g), 2-mercapto-5-trifluoromethylpyridine (1.9 g) and potassium carbonate (2.0 g) in N,N-dimethylformamide (40 ml) was heated at 90°C for 4.5 hours with stirring. The reaction mixture was concentrated under reduced pressure and the residue was triturated with water. The precipitates were collected by filtration, washed with water and dried in vacuo to give 2-acetylamino-5-(5-trifluoromethylpyridin-2-ylthio)thiazole (3.2 g, yield : 100%).
mp : 165-170°C (dec.)
IR (Nujol) : 3175, 1695, 1640, 1600, 1565, 1330 cm⁻¹
NMR (DMSO-d₆, 200MHZ, ppm) : 2.19 (3H, s), 7.21 (1H, d, J=12Hz), 7.83 (1H, s), 8.04-8.15 (1H, m), 8.18 (1H, br s), 12.53 (1H, s)
Mass : M⁺¹ 321, M 320, M 319, m/e 277, 235, 191

### Example 51

A mixture of 2-acetylamino-5-(5-trifluoromethylpyridin-2-ylthio)thiazole (3.2 g) in a mixture of ethanol (60 ml), tetrahydrofuran (30 ml) and aqueous 6N-hydrochloric acid (10 ml) was refluxed for 3 hours with stirring. The reaction mixture was concentrated under reduced pressure and the residue was dissolved in water. The solution was adjusted to pH 8.5 using aqueous sodium bicarbonate and extracted with mixture of tetrahydrofuran and ethyl acetate (1:1) and dried over magnesium sulfate. The solvent was concentrated under reduced pressure to give solid. The solid was subjected to column chromatography on silica gel (silica gel 60, 70-230 mesh; Merck : 150 g) and eluted with a mixture of chloroform and methanol (10:1). The fractions containing the objective compound were combined and concentrated under reduced pressure to give 2-amino-5-(5-trifluoromethylpyridin-2-ylthio)thiazole (2.1 g, yield : 75.8%).
mp : 135-138°C
IR (Nujol) : 3400, 3300, 3100, 1640, 1600, 1560, 1520, 1330 cm⁻¹
NMR (DMSO-d₆, 200MHZ, ppm) : 7.26 (1H, d, J=8Hz), 7.33 (1H, s), 7.66 (2H, s), 7.81 (1H, d, J=8Hz), 8.80 (1H, s)
Mass : M⁺¹ 278, M 277, m/e 235, 191, 146, 131

### Example 52

To a solution of 2-amino-5-(5-trifluoromethylpyridin-2-ylthio)thiazole (0.6 g) in dichloromethane (20 ml) was added portionwise 3-chloroperbenzoic acid (0.6 g) at 5°C with stirring. The mixture was stirred at 5°C for 3 hours. The reaction mixture was washed with aqueous sodium bicarbonate and dried over magnesium sulfate. The solvent was concentrated under reduced pressure to give solid. The solid was subjected to column chromatography on silica gel (silica gel 60, 70-230 mesh; Merck : 30 g) and eluted with a mixture of chloroform and methanol (10:1). The fractions containing the objective compound were combined and concentrated under reduced pressure to give 2-amino-5-(5-trifluoromethylpyridin-2-ylsulfinyl)-thiazole (0.52 g, yield : 81.9%).
mp : 144-145°C
NMR (DMSO-d₆, 200MHZ, ppm) : 7.82 (1H, s), 7.92 (2H, s), 8.21 (1H, d, J=8Hz), 8.56 (1H, d, J=8Hz), 9.08 (1H, s)
Mass : M 293, m/e 277, 245, 226, 179, 147

### Example 53

A mixture of 2-acetylamino-5-bromothiazole (2.2 g), 4-amino-2-mercaptopyrimidine (1.3 g) and potassium carbonate (2.0 g) in N,N-dimethylformamide (50 ml) was heated at 90°C for 2 hours with stirring. The reaction mixture was concentrated under reduced pressure and the residue was triturated with water. The precipitation was collected by filtration, washed with water and dried in vacuo to give solid. The solid was subjected to column chromatography on silica gel (silica gel 60, 70-230 mesh; Merck : 200 g) and eluted with a mixture of chloroform and methanol (10:1). The fractions containing the objective compound were combined and concentrated under reduced pressure to give 2-acetylamino-5-(4-aminopyrimidin-2-ylthio)thiazole (1.3 g, yield : 48.7%).
mp : 255-258°C (dec.)
IR (Nujol) : 3400, 3350, 3200, 1692, 1650, 1585, 1325, 1300 cm⁻¹
NMR (DMSO-d₆, 200MHZ, ppm) : 2.16 (3H, s), 6.17 (1H, d, J=6Hz), 7.02 (2H, s), 7.59 (1H, s), 7.85 (1H, d, J=6Hz), 12.31 (1H, s)
Mass : M⁺¹ 268, M 267, m/e 225, 205, 183

### Example 54

Starting from 2-acetylamino-5-bromothiazole, 2-acetylamino-5-(4-hydroxypyrimidin-2-ylthio)thiazole (0.35 g, yield : 28.8%) was obtained according to a similar manner to that of Example 41.
IR (Nujol) : 3150, 1665, 1565, 1535, 1300, 1275, 1230 cm⁻¹
NMR (DMSO-d₆, 200MHZ, ppm) : 2.18 (3H, s), 6.25 (1H, d, J=6Hz), 7.70 (1H, s), 7.93 (1H, d, J=6Hz), 12.0-12.6 (2H, m)
Mass : M⁺¹ 269, M 268, m/e 259, 197, 135

### Example 55

A mixture of 2-acetylamino-5-(4-hydroxypyrimidin-2-ylthio)thiazole (3.7 g) in a mixture of ethanol (100 ml), tetrahydrofuran (40 ml) and aqueous 6N-hydrochloric acid (20 ml) was refluxed for 6.5 hours with stirring. The reaction mixture was concentrated under reduced pressure and the residue was dissolved in water. The solution was adjusted to pH 8.5 using aqueous sodium bicarbonate. The precipitates were collected by filtration, washed with water and dried in vacuo to give solid. The solid was subjected to column chromatography on silica gel (silica gel 60, 70-230 mesh; Merck : 250 g) and eluted with a mixture of chloroform and methanol (10:1). The fractions containing the objective compound were combined and concentrated under reduced pressure to give 2-amino-5-(4-hydroxypyrimidin-2-ylthio)thiazole (0.45 g, yield : 14.5%).
mp : 210-220°C (dec.)
IR (Nujol) : 3450, 3350, 3125, 1675, 1510,1230 cm⁻¹
NMR (DMSO-d₆ 200MHZ, ppm) : 5.45 (1H, d, J=7Hz), 7.30-7.40 (3H, m), 7.64 (1H, m)
Mass : m/e 220, 205, 132, 112

### Example 56

Starting from 2-acetylamino-5-bromothiazole, 2-acetylamino-5-(4-methylpyrimidin-2-ylthio)thiazole (2.89 g, yield : 48.0%) was obtained according to a similar manner to that of Example 67.
mp : 210°C (dec.)
IR (Nujol) : 3170, 1720, 1695, 1575, 1555, 1335 cm⁻¹
NMR (DMSO-d₆, 200MHZ, ppm) : 2.19 (3H, s), 2.39 (3H, s), 7.16 (1H, d, J=5Hz), 7.70 (1H, s), 8.46 (1H, d, J=5Hz), 12.38 (1H, s)
Mass : M⁺ 268, M⁺¹ 267, M 266, m/e 224, 182, 165

### Example 57

Starting from 2-acetylamino-5-(4-methylpyrimidin-2-ylthio)thiazole, 2-amino-5-(4-methylpyrimidin-2-ylthio)-thiazole (0.40 g, yield : 16.4%) was obtained by refluxing with stirring in a mixture of ethanol and aqueous 6 N hydrochlorid acid. The reaction mixture was concentrated under reduced pressure and the residue was dissolved in water. The solution was adjusted to pH 10 using aqueous sodium hydroxide under cooling. The precipitates were collected by filtration, washed with water and recrystallized from ethanol to give the desired compound.
mp : 158-159°C
IR (Nujol) : 3430, 3280, 3100, 1620, 1565, 1520, 1490, 1330, 1210 cm⁻¹
NMR (DMSO-d₆, 200MHZ, ppm) : 2.39 (3H, s), 7.14 (1H, d, J=5Hz), 7.15 (1H, s), 7.43 (2H, s), 8.47 (1H, d, J=5Hz)
Mass : M⁺ 226, M⁺¹ 225, M 224, m/e 182, 138

| Analysis Calcd. for C₈H₈N₄S₂ | | | |
|---|---|---|---|
| | C 42.84, | H 3.59, | N 24.99 |
| Found : | C 42.81, | H 3.50, | N 24.86 |

### Example 58

To a solution of 2-amino-5-(2-pyrimidinylthio)-thiazole (1.0 g) in a pyridine (20 ml) was dropwise added methanesulfonyl chloride (0.8 ml) at 5°C with stirring. The mixture was stirred at room temperature for 24 hours. The reaction mixture was concentrated under reduced pressure and water was added to this residue. The mixture was extracted with a mixture of tetrahydrofuran and ethyl acetate (1:1), washed with aqueous saturated sodium chloride and dried over magnesium sulfate. The solution was concentrated under reduced pressure to give solid. The solid was recrystallized from 50% ethanol to give 2-methanesulfonylamino-5-(2-pyrimidinylthio)-thiazole (0.60 g, yield : 43.8%).
mp : 200°C (dec.)
IR (Nujol) : 3120, 1585, 1545, 1440, 1305, 1140 cm⁻¹
NMR (DMSO-d₆, 200MHZ, ppm) : 2.98 (3H, s), 7.35 (1H, t, d=7Hz), 7.76 (1H, s), 8.71 (2H, d, J=7Hz), 12.89 (1H, br s)
Mass : M⁺ 290, M⁺¹ 289, M 288, m/e 209, 168

| Analysis Calcd. for C₈H₈N₄O₂S₃ | | | |
|---|---|---|---|
| | C 33.32, | H 2.80, | N 19.43 |
| Found : | C 33.04, | H 2.74, | N 19.06 |

### Example 59

Starting from 2-amino-5-(2-pyrimidinylthio)thiazole, 2-amino-5-(2-pyrimidinylsulfinyl)thiazole (0.52 g, yield : 32.2%) was obtained according to a similar manner to that of Example 40.
mp : 206°C (dec.)
IR (Nujol) : 3300, 3200, 1615, 1565, 1545, 1520, 1230, 1150 cm⁻¹
NMR (DMSO-d₆, 200MHZ, ppm) : 7.67 (1H, t, J=5Hz), 7.73 (1H, s), 7.87 (2H, s), 8.99 (2H, d, J=5Hz)
Mass : M 226, m/e 210, 178, 168, 147

| Analysis Calcd. for C₇H₆N₄OS₂ | | | |
|---|---|---|---|
| | C 37.16, | H 2.67, | N 24.76 |
| Found : | C 36.78, | H 2.62, | N 24.62 |

### Example 60

Starting from 2-amino-5-(2-pyrimidinylthio)thiazole, 2-amino-5-(2-pyrimidinylsulfonyl)thiazole (0.464 g, yield : 8.1%) was obtained according to a similar manner to that of Example 49.
mp : 214°C (dec.)
IR (Nujol) : 3400, 3100, 1615, 1570, 1515, 1335, 1210, 1140 cm⁻¹
NMR (DMSO-d₆, 200MHZ, ppm) : 7.73 (1H, s), 7.80 (1H, t, d=5Hz), 8.23 (2H, s), 9.05 (2H, d, J=5Hz)
Mass : M⁺ 244, M⁺¹ 243, M 242, m/e 178, 136

## Claims (Claims for the following Contracting State(s): DE, AT, GB, FR, BE, IT, NL, CH, LI, LU, SE, DK)

1. A compound of the formula : 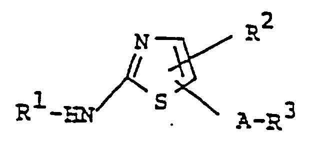 wherein
R¹ is hydrogen, (C₁-C₆)alkanoyl, (C₁-C₆)alkanesulfonyl or benzoyl which may be substituted with halogen,
R is hydrogen, (C₁-C₆)alkyl, hydroxy(C₁-C₆)alkyl, halogen or carboxy,
A is -C(=NOR⁴)- [wherein R⁴ is C₁-C₆ alkyl], 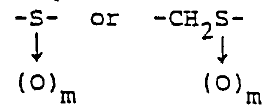 [wherein m is an integer of 0, 1 or 2], and
R³ is phenyl, toluolyl, xylyl, cumenyl or naphthyl which may be substituted with halogen, hydroxy, (C₁-C₆)alkoxy, nitro, amino or acylamino; or
pyridyl, pyrimidinyl, imidazolyl, tetrazolyl or thiadiazolyl, each of which may be substituted with (C₁-C₆)alkyl, amino, hydroxy or halo(C₁-C₆)alkyl, provided that R³ is pyridyl, pyrimidinyl, imidazolyl, tetrazolyl or thiadiazolyl, each of which may be substituted with (C₁-C₆)alkyl, amino, hydroxy or halo(C₁-C₆)alkyl when A is 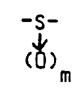 [wherein m is an integer of 0, 1 or 2],
and pharmaceutically acceptable salts thereof.

2. A compound of claim 1, wherein
R¹ is hydrogen.

3. A compound of claim 2, wherein
R is hydrogen.

4. A compound of claim 3, wherein
A is 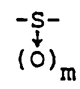 [wherein m is 0, 1 or 2].

5. A compound of claim 4, wherein
R³ is pyridyl, pyrimidinyl, imidazolyl, tetrazolyl or thiadiazolyl.

6. A process for preparing a compound of the formula : 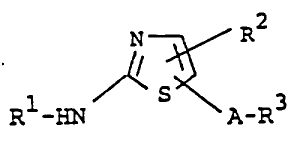 wherein
R¹ is hydrogen, (C₁-C₆)alkanoyl, (C₁-C₆)alkanesulfonyl or benzoyl which may be substituted with halogen,
R is hydrogen, (C₁-C₆)alkyl, hydroxy(C₁-C₆)alkyl, halogen or carboxy,
A is -C(=NOR⁴)- [wherein R⁴ is (C₁-C₆) alkyl], 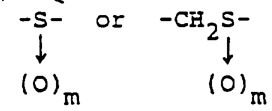 [wherein m is an integer of 0, 1 or 2], and
R³ is phenyl, toluolyl, xylyl, cumenyl or naphthyl which may be substituted with halogen, hydroxy, (C₁-C₆)alkoxy, nitro, amino or acylamino; or
pyridyl, pyrimidinyl, imidazolyl, tetrazolyl or thiadiazolyl, each of which may be substituted with (C₁-C₆)alkyl, amino, hydroxy or halo(C₁-C₆)alkyl, provided that R³ is pyridyl, pyrimidinyl, imidazolyl, tetrazolyl or thiadiazolyl, each of which may be substituted with (C₁-C₆)alkyl, amino, hydroxy or halo(C₁-C₆)alkyl when A is 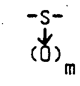 [wherein m is an integer of 0, 1 or 2],
or its salt,
which comprises
(1) deacylating a compound of the formula : 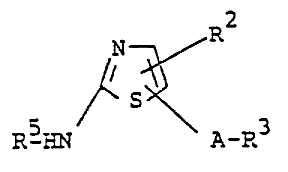 or its salt to give a compound of the formula : 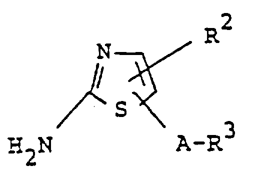 or its salt, in which R, R³ and A are each as defined above,
R⁵ is acyl which may be substituted with halogen, or
(2) reacting a compound of the formula : 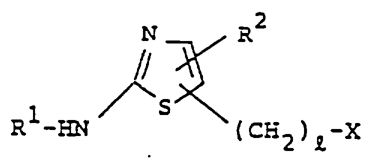 or its salt with a compound of the formula :
R³ - SH
or its salt to give a compound of the formula : 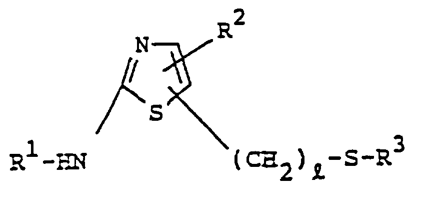 or its salt, in which R¹, R and R³ are each as defined above,
X is halogen and ℓ is 0 or 1, or
(3) reducing a compound of the formula : 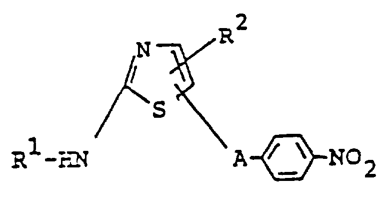 or its salt to give a compound of the formula : 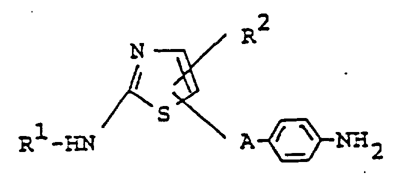 or its salt, in which R¹, R and A are each as defined above,
or
(4) subjecting a compound of the formula : 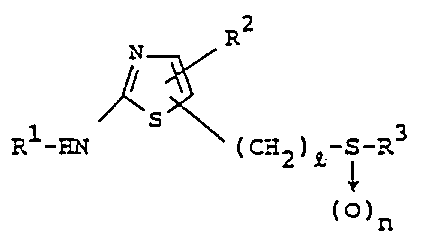 or its salt to oxidation to give a compound of the formula : 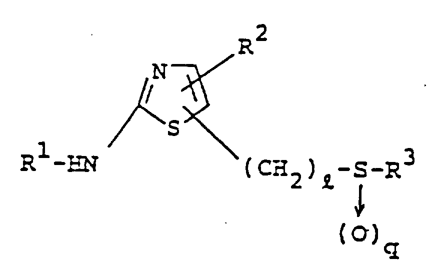 or its salt, in which R¹, R, R³ and ℓ are each as defined above,
n is 0 or 1 and q is 1 or 2, provided that q is 2 when n is 1, or
(5) halogenating a compound of the formula : 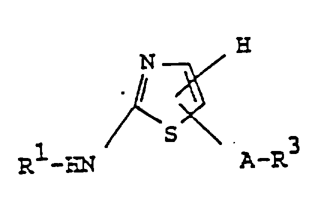 or its salt to give a compound of the formula : 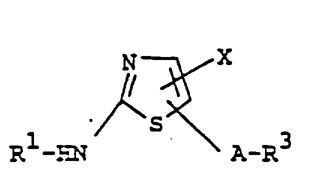 or its salt, in which R¹, R³, A and X are each as defined above,
or
(6) acylating a compound of the formula : 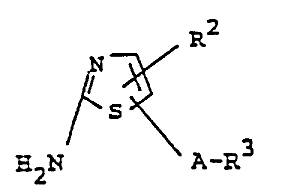 or its reactive derivative at the amino group or a salt thereof to give a compound of the formula : 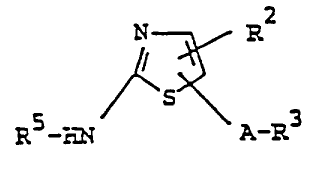 or its salt, in which R, R³, R⁵ and A are each as defined above,
or
( 7) acylating a compound of the formula : 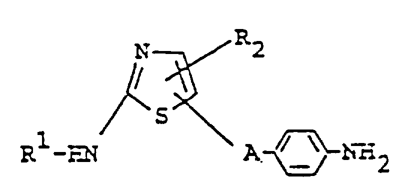 or its reactive derivative at the amino group or a salt thereof to give a compound of the formula : 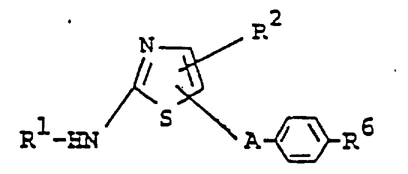 or its salt, in which R¹, R and A are each as defined above,
R⁶ is acylamino, or
(8) reacting a compound of the formula : 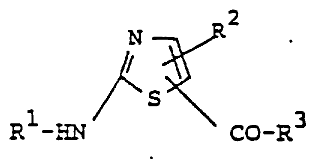 or its salt with a compound of the formula :
H₂NOR⁴
or its salt to give a compound of the formula : 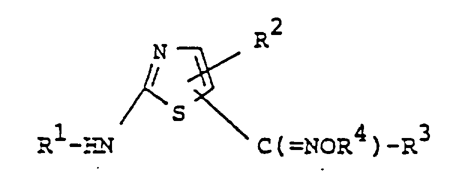 or its salt, in which R¹, R, R³ and R⁴ are each as defined above.

7. A pharmaceutical composition which comprises, as an active ingredient, a compound of claim 1 or a pharmaceutically acceptable salt thereof in admixture with pharmaceutically acceptable carriers.

8. Use of a compound of claim 1 or a pharmaceutically acceptable salt thereof for the manufacture of a medicament for therapeutic treatment of rheumatism, nephritis, thrombocytopenia, tumor and side effects of an antitumor agent.

9. A compound of claim 1 or a pharmaceutically acceptable salt thereof for use as a medicament.

## Claims (Claims for the following Contracting State(s): ES)

1. A process for preparing a compound of the formula : 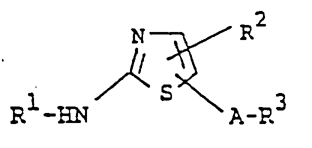 wherein
R¹ is hydrogen, (C₁-C₆)alkanoyl, (C₁-C₆)alkanesulfonyl or benzoyl which may be substituted with halogen,
R is hydrogen, (C₁-C₆)alkyl, hydroxy(C₁-C₆)alkyl, halogen or carboxy,
A is -C(=NOR⁴)- [wherein R⁴ is (C₁-C₆) alkyl], 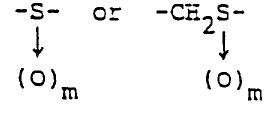 [wherein m is an integer of 0, 1 or 2], and
R³ is phenyl, toluolyl, xylyl, cumenyl or naphthyl which may be substituted with halogen, hydroxy, (C₁-C₆)alkoxy, nitro, amino or acylamino; or
pyridyl, pyrimidinyl, imidazolyl, tetrazolyl or thiadiazolyl, each of which maybe substituted with (C₁-C₆)alkyl, amino, hydroxy or halo(C₁-C₆)alkyl, provided that R³ is pyridyl, pyrimidinyl, imidazolyl, tetrazolyl or thiadiazolyl, each of which may be substituted with (C₁-C₆)alkyl, amino, hydroxy or with (C₁-C₆)alkyl, amino, hydroxy or halo(C₁-C₆)alkyl when A is 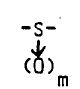 [wherein m is an integer of 0, 1 or 2],
or its salt,
which comprises
(1) deacylating a compound of the formula : 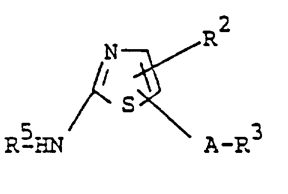 or its salt to give a compound of the formula : 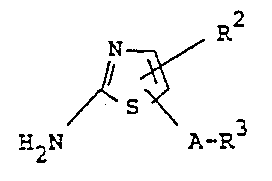 or its salt, in which R, R³ and A are each as defined above,
R⁵ is acyl which may be substituted with halogen, or
(2) reacting a compound of the formula : 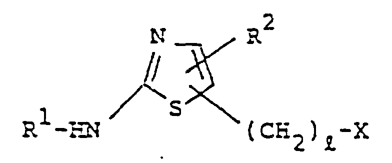 or its salt with a compound of the formula :
R³ - SH
or its salt to give a compound of the formula : 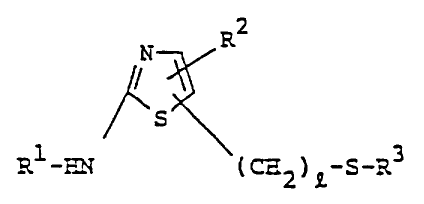 or its salt, in which R¹, R and R³ are each as defined above,
X is halogen and ℓ is 0 or 1, or
(3) reducing a compound of the formula : 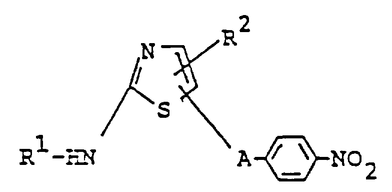 or its salt to give a compound of the formula : 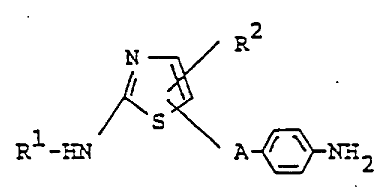 or its salt, in which R¹, R and A are each as defined above,
or
(4) subjecting a compound of the formula : 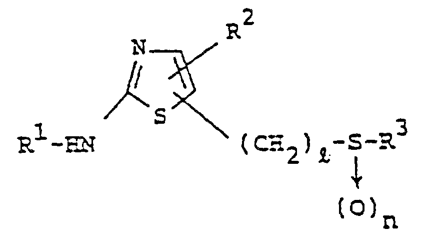 or its salt to oxidation to give a compound of the formula : 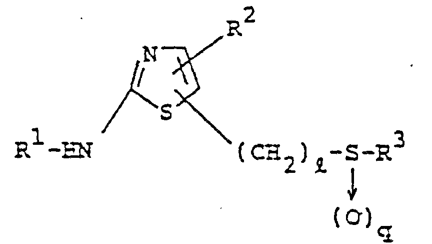 or its salt, in which R¹, R, R³ and ℓ are each as defined above,
n is 0 or 1 and q is 1 or 2, provided that q is 2 when n is 1, or
(5) halogenating a compound of the formula : 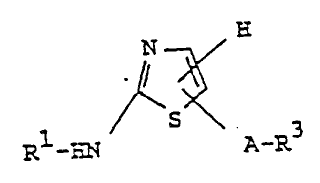 or its salt to give a compound of the formula : 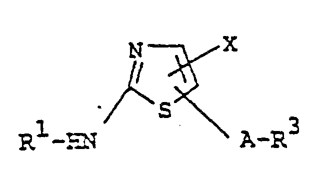 or its salt, in which R¹, R³, A and X are each as defined above,
or
(6) acylating a compound of the formula : 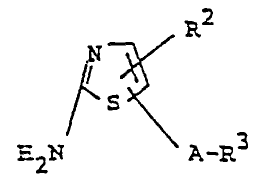 or its reactive derivative at the amino group or a salt thereof to give a compound of the formula : 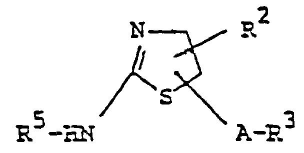 or its salt, in which R, R³, R⁵ and A are each as defined above,
or
(7) acylating a compound of the formula : 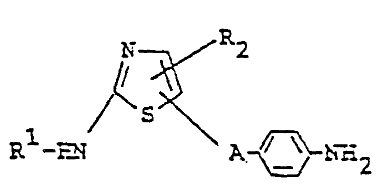 or its reactive derivative at the amino group or a salt thereof to give a compound of the formula : 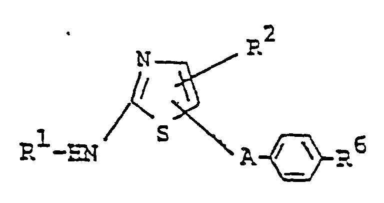 or its salt, in which R¹, R and A are each as defined above,
R⁶ is acylamino, or
(8) reacting a compound of the formula : 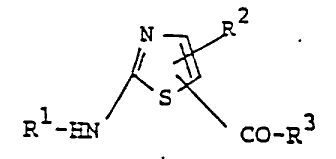 or its salt with a compound of the formula :
H₂NOR⁴
or its salt to give a compound of the formula : 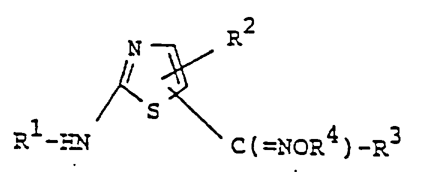 or its salt, in which R¹, R, R³ and R⁴ are each as defined above.

## Claims (Claims for the following Contracting State(s): GR)

1. A process for preparing a compound of the formula : 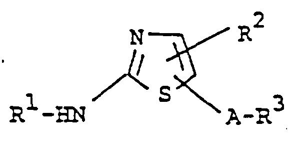 wherein
R¹ is hydrogen,(C₁-C₆)alkanoyl, (C₁-C₆)alkanesulfonyl or benzoyl which may be substituted with halogen,
R is hydrogen, (C₁-C₆)alkyl, hydroxy(C₁-C₆)alkyl, halogen or carboxy,
A is -C(=NOR⁴)- [wherein R⁴ is (C₁-C₆) alkyl], 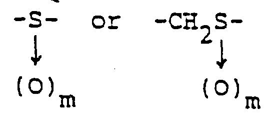 [wherein m is an integer of 0, 1 or 2], and
R³ is phenyl, toluolyl, xylyl, cumenyl or naphthyl which may be substituted with halogen, hydroxy, (C₁-C₆)alkoxy, nitro, amino or acylamino; or
pyridyl, pyrimidinyl, imidazolyl, tetrazolyl or thiadiazolyl, each of which may be substituted with (C₁-C₆)alkyl, amino, hydroxy or halo(C₁-C₆)alkyl, provided that R³ is pyridyl, pyrimidinyl, imidazolyl, tetrazolyl or thiadiazolyl, each of which may be substituted with (C₁-C₆)alkyl, amino, hydroxy or halo(C₁-C₆)alkyl when A is 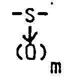 [wherein m is an integer of 0, 1 or 2],
or its salt,
which comprises
(1) deacylating a compound of the formula : 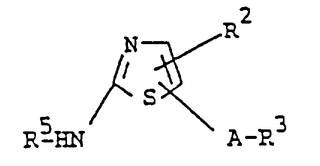 or its salt to give a compound of the formula : 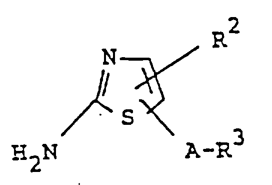 or its salt, in which R, R³ and A are each as defined above,
R⁵ is acyl which may be substituted with halogen, or
(2) reacting a compound of the formula : 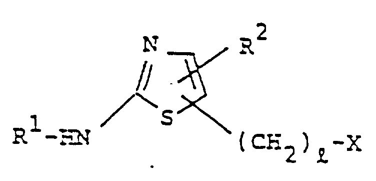 or its salt with a compound of the formula :
R³ - SH
or its salt to give a compound of the formula : 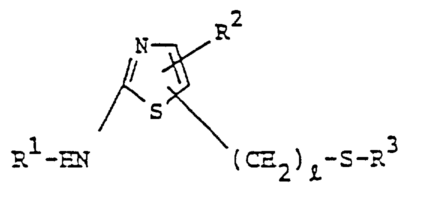 or its salt, in which R¹, R and R³ are each as defined above,
X is halogen and ℓ is 0 or 1, or
(3) reducing a compound of the formu.la : 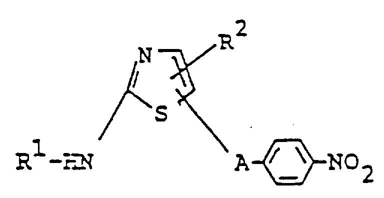 or its salt to give a compound of the formula : 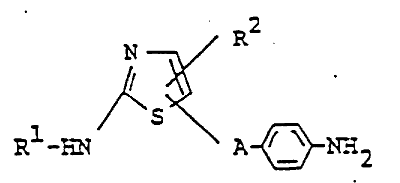 or its salt, in which R¹, R and A are each as defined above,
or
(4) subjecting a compound of the formula : 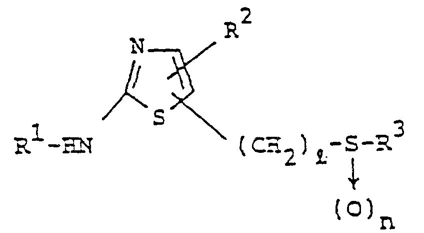 or its salt to oxidation to give a compound of the formula : 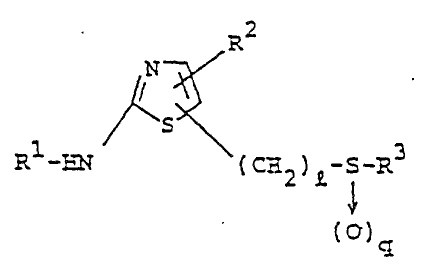 or its salt, in which R¹, R, R³ and l are each as defined above,
n is 0 or 1 and q is 1 or 2, provided that q is 2 when n is 1, or
(5) halogenating a compound of the formula : 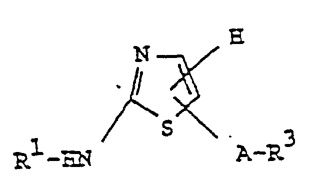 or its salt to give a compound of the formula : 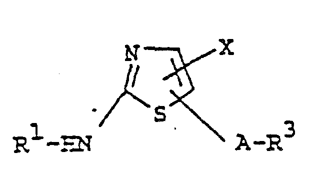 or its salt, in which R¹, R³, A and X are each as defined above,
or
(6) acylating a compound of the formula : 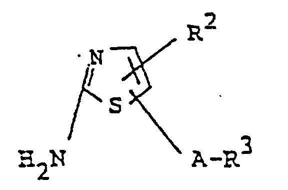 or its reactive derivative at the amino group or a salt thereof to give a compound of the formula : 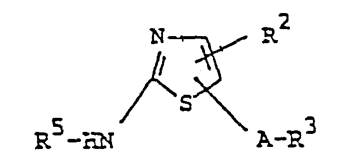 or its salt, in which R, R³, R⁵ and A are each as defined above,
or
(7) acylating a compound of the formula : 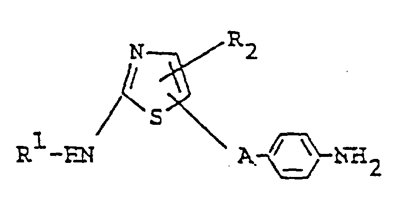 or its reactive derivative at the amino group or a salt thereof to give a compound of the formula : 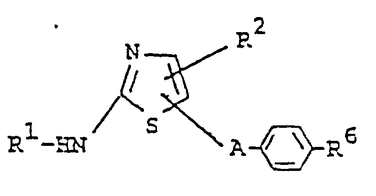 or its salt, in which R¹, R and A are each as defined above,
R⁶ is acylamino, or
(8) reacting a compound of the formula : 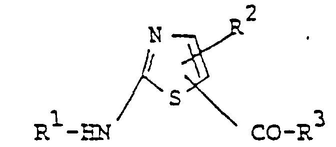 or its salt with a compound of the formula :
H₂NOR⁴
or its salt to give a compound of the formula : 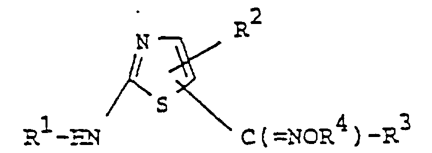 or its salt, in which R¹, R, R³ and R⁴ are each as defined above.

2. A modification of the process claimed in claim 1 which is characterized by bringing a compound of the formula 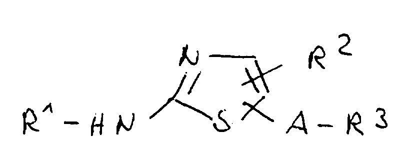 wherein R¹, R, A, and R³ are defined as in claim 1, or a non-toxic salt thereof, produced by a process claimed in claim 1, into pharmaceutically acceptable form by admixture or presentation of said compound with a pharmaceutically acceptable diluent or carrier.

## Patentansprüche (Patentansprüche für folgende(n) Vertragsstaat(en): DE, AT, GB, FR, BE, IT, NL, CH, LI, LU, SE, DK)

1. Verbindung der Formel: 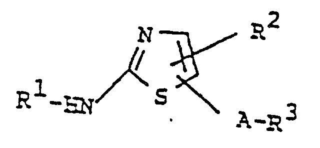 worin bedeuten:
R¹ Wasserstoff, (C₁₋₆)-Alkanoyl, (C₁₋₆)-Alkansulfonyl oder Benzoyl, die durch Halogen substituiert sein können,
R Wasserstoff, (C₁₋₆)-Alkyl, Hydroxy(C₁₋₆)-alkyl, Halogen oder Carboxy,
A -C(=NOR⁴) -, [worin R⁴ steht für (C₁₋₆)-Alkyl], 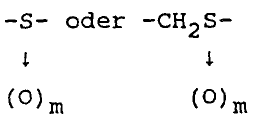 [worin m steht für die ganze Zahl 0, 1 oder 2], und
R³ Phenyl, Toluolyl, Xylyl, Cumenyl oder Naphthyl, die substituiert sein können durch Halogen, Hydroxy, (C₁₋ ₆)-Alkoxy, Nitro, Amino oder Acylamino; oder Pyridyl, Pyrimidinyl, Imidazolyl, Tetrazolyl oder Thiadiazolyl, von denen jedes substituiert sein kann durch (C₁₋₆)-Alkyl, Amino, Hydroxy oder Halogen(C₁₋ ₆)-alkyl, mit der Maßgabe, daß R³ für Pyridyl, Pyrimidinyl, Imidazolyl, Tetrazolyl oder Thiadiazolyl steht, von denen jedes substituiert sein kann durch (C₁₋₆)-Alkyl, Amino, Hydroxy oder Halogen(C₁₋₆)-alkyl, wenn A 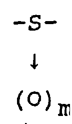 bedeutet [worin m die ganze Zahl 0, 1 oder 2 darstellt].
und ihre pharmazeutisch akzeptablen Salze.

2. Verbindung nach Anspruch 1, worin R¹ für Wasserstoff steht.

3. Verbindung nach Anspruch 2, worin R für Wasserstoff steht.

4. Verbindung nach Anspruch 3, worin A für 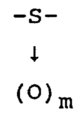 steht (worin m = 0, 1 oder 2).

5. Verbindung nach Anspruch 4, worin R³ für Pyridyl, Pyrimidinyl, Imidazolyl, Tetrazolyl oder Thiadiazolyl steht.

6. Verfahren zur Herstellung einer Verbindung der Formel: 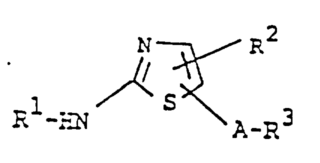 worin bedeuten:
R¹ Wasserstoff, (C₁₋₆)-Alkanoyl, (C₁₋₆)-Alkansulfonyl oder Benzoyl, die durch Halogen substituiert sein können,
R Wasserstoff, (C₁₋₆)-Alkyl, Hydroxy(C₁₋₆)-alkyl, Halogen oder Carboxy,
A -C(=NOR⁴)-, [worin R⁴ steht für (C₁₋₆)-Alkyl], 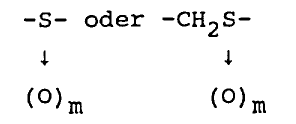 [worin m steht für die ganze Zahl 0, 1 oder 2], und
R³ Phenyl, Toluolyl, Xylyl, Cumenyl oder Naphthyl, die substituiert sein können durch Halogen, Hydroxy, (C₁₋ ₆)-Alkoxy, Nitro, Amino oder Acylamino; oder Pyridyl, Pyrimidinyl, Imidazolyl, Tetrazolyl oder Thiadiazolyl, von denen jedes substituiert sein kann durch (C₁₋₆)-Alkyl, Amino, Hydroxy oder Halogen(C₁₋ ₆)-alkyl, mit der Maßgabe, daß R³ für Pyridyl, Pyrimidinyl, Imidazolyl, Tetrazolyl oder Thiadiazolyl steht, von denen jedes substituiert sein kann durch (C₁₋₆)-Alkyl, Amino, Hydroxy oder Halogen(C₁₋₆)-alkyl, wenn A 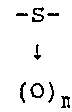 bedeutet [worin m die ganze Zahl 0, 1 oder 2 darstellt].
oder ihres Salzes, das umfaßt
1) die Deacylierung einer Verbindung der Formel 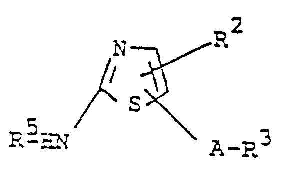 oder ihres Salzes unter Bildung einer Verbindung der Formel 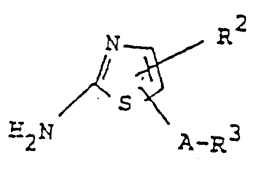 oder ihres Salzes,
worin R, R³ und A jeweils wie oben definiert sind und R⁵ für Acyl steht, das durch Halogen substituiert sein kann, oder
2) die Umsetzung einer Verbindung der Formel 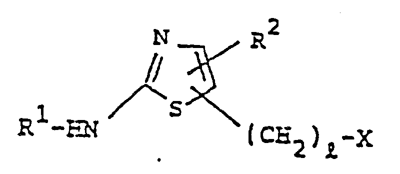 oder ihres Salzes mit einer Verbindung der Formel
R³ - SH
oder ihrem Salz unter Bildung einer Verbindung der Formel 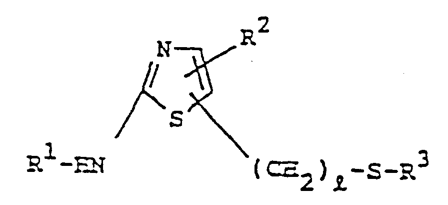 oder ihres Salzes,
worin R¹, R und R³ jeweils wie oben definiert sind und X für Halogen und 1 für 0 oder 1 stehen, oder
3) die Reduktion einer Verbindung der Formel 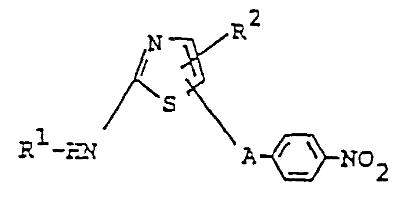 oder ihres Salzes unter Bildung einer Verbindung der Formel 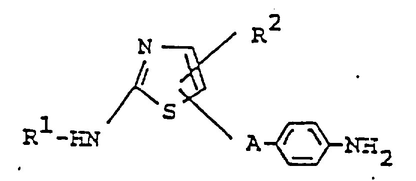 oder ihres Salzes,
worin R¹, R und A jeweils wie oben definiert sind, oder
4) die Oxidation einer Verbindung der Formel 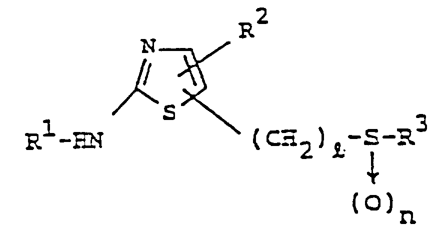 oder ihres Salzes unter Bildung einer Verbindung der Formel 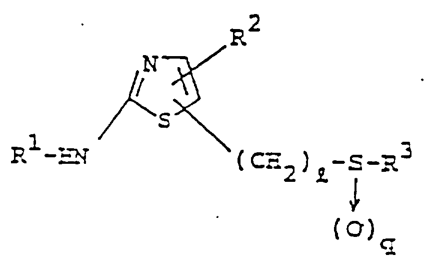 oder ihres Salzes,
worin R¹, R, R³ und 1 jeweils wie oben definiert sind, n für 0 oder 1 und q für 1 oder 2 stehen, mit der Maßgabe, daß q = 2, wenn n = 1, oder
5) die Halogenierung einer Verbindung der Formel 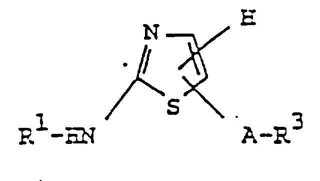 oder ihres Salzes unter Bildung einer Verbindung der Formel 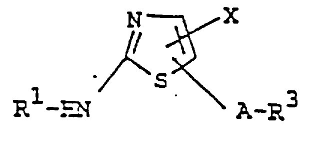 oder ihres Salzes,
worin R¹, R³, A und X jeweils wie oben definiert sind, oder
6) die Acylierung einer Verbindung der Formel 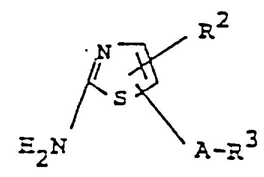 oder ihres reaktionsfähigen Derivats an der Aminogruppe oder eines Salzes derselben unter Bildung einer Verbindung der Formel 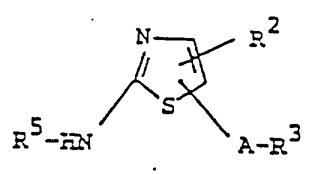 oder ihres Salzes,
worin R, R³, R⁵ und A jeweils wie oben definiert sind, oder
7) die Acylierung einer Verbindung der Formel 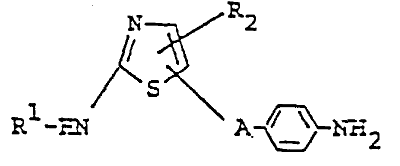 oder ihres reaktionsfähigen Derivats an der Aminogruppe oder eines Salzes derselben unter Bildung einer Verbindung der Formel 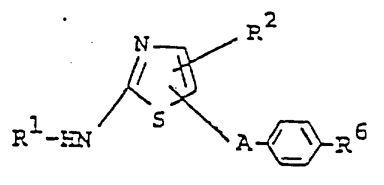 oder ihres Salzes,
worin R¹, R und A jeweils wie oben definiert sind und R⁶ für Acylamino steht, oder
8) die Umsetzung einer Verbindung der Formel 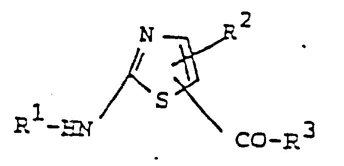 oder ihres Salzes mit einer Verbindung der Formel
H₂NOR⁴
oder ihrem Salz unter Bildung einer Verbindung der Formel 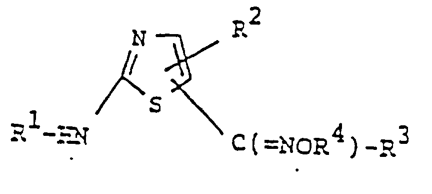 oder ihres Salzes,
worin R¹, R, R³ und R⁴ jeweils wie oben definiert sind.

7. Pharmazeutische Zusammensetzung, die umfaßt als aktiven Bestandteil (Wirkstoff) eine Verbindung nach Anspruch 1 oder ein pharmazeutisch akzeptables Salz derselben im Gemisch mit pharmazeutisch akzeptablen Trägern.

8. Verwendung einer Verbindung nach Anspruch 1 oder eines pharmazeutisch akzeptablen Salzes derselben für die Herstellung eines Arzneimittels für die therapeutische Behandlung von Rheumatismus, Nephritis, Thrombozytopenie, Tumor und der Nebenwirkungen eines Antitumormittels.

9. Verbindung nach Anspruch 1 oder ein pharmazeutisch akzeptables Salz derselben für die Verwendung als Arzneimittel.

## Patentansprüche (Patentansprüche für folgende(n) Vertragsstaat(en): ES)

1. Verfahren zur Herstellung einer Verbindung der Formel: 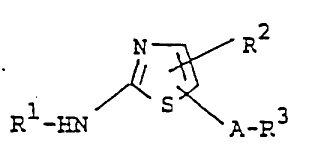 worin bedeuten:
R¹ Wasserstoff, (C₁₋₆)-Alkanoyl, (C₁₋₆)-Alkansulfonyl oder Benzoyl, die durch Halogen substituiert sein können,
R Wasserstoff, (C₁₋₆)-Alkyl, Hydroxy(C₁₋₆)-alkyl, Halogen oder Carboxy,
A -C(=NOR⁴)-, [worin R⁴ steht für (C₁₋₆)-Alkyl], 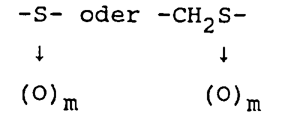 [worin m steht für die ganze Zahl 0, 1 oder 2], und
R³ Phenyl, Toluolyl, Xylyl, Cumenyl oder Naphthyl, die substituiert sein können durch Halogen, Hydroxy, (C₁₋ 6)-Alkoxy, Nitro, Amino oder Acylamino; oder Pyridyl, Pyrimidinyl, Imidazolyl, Tetrazolyl oder Thiadiazolyl, von denen jedes substituiert sein kann durch (C₁₋₆)-Alkyl, Amino, Hydroxy oder Halogen(C₁₋ ₆)-alkyl, mit der Maßgabe, daß R³ für Pyridyl, Pyrimidinyl, Imidazolyl, Tetrazolyl oder Thiadiazolyl steht, von denen jedes substituiert sein kann durch (C₁₋₆)-Alkyl, Amino, Hydroxy oder Halogen (C₁₋₆)-alkyl, wenn A 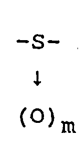 bedeutet [worin m die ganze Zahl 0, 1 oder 2 darstellt].
oder ihres Salzes, das umfaßt
1) die Deacylierung einer Verbindung der Formel 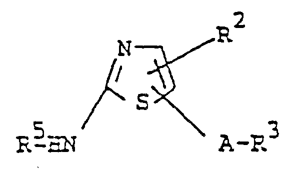 oder ihres Salzes unter Bildung einer Verbindung der Formel 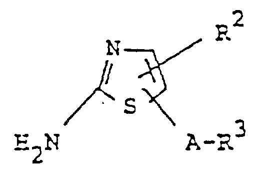 oder ihres Salzes,
worin R, R³ und A jeweils wie oben definiert sind und R⁵ für Acyl steht, das durch Halogen substituiert sein kann, oder
2) die Umsetzung einer Verbindung der Formel 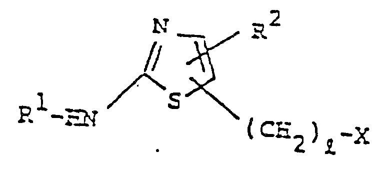 oder ihres Salzes mit einer Verbindung der Formel
R³ - SH
oder ihrem Salz unter Bildung einer Verbindung der Formel 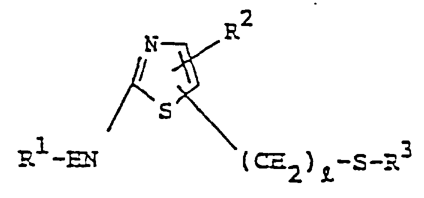 oder ihres Salzes,
worin R¹, R und R³ jeweils wie oben definiert sind und X für Halogen und 1 für 0 oder 1 stehen, oder
3) die Reduktion einer Verbindung der Formel 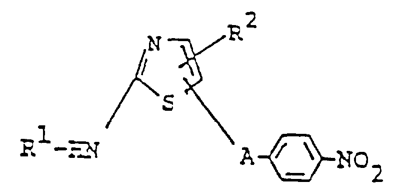 oder ihres Salzes unter Bildung einer Verbindung der Formel 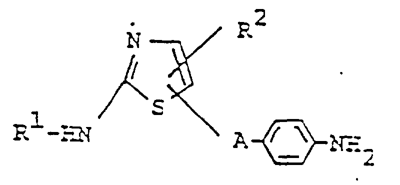 oder ihres Salzes,
worin R¹, R und A jeweils wie oben definiert sind, oder
4) die Oxidation einer Verbindung der Formel 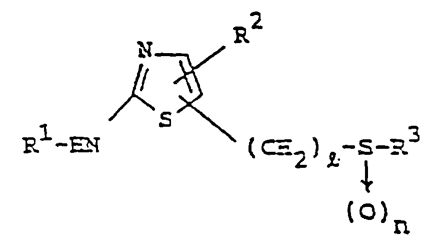 oder ihres Salzes unter Bildung einer Verbindung der Formel 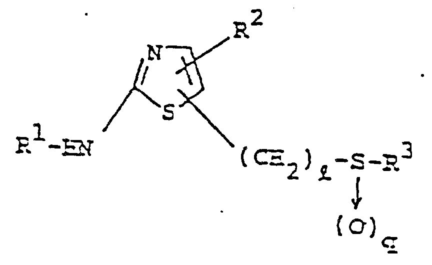 oder ihres Salzes,
worin R¹, R, R³ und 1 jeweils wie oben definiert sind, n für 0 oder 1 und q für 1 oder 2 stehen, mit der Maßgabe, daß q = 2, wenn n = 1, oder
5) die Halogenierung einer Verbindung der Formel 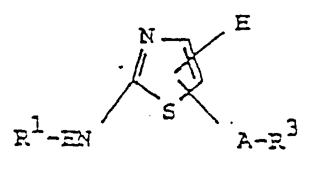 oder ihres Salzes unter Bildung einer Verbindung der Formel 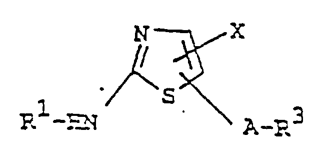 oder ihres Salzes,
worin R¹, R³, A und X jeweils wie oben definiert sind, oder
6) die Acylierung einer Verbindung der Formel 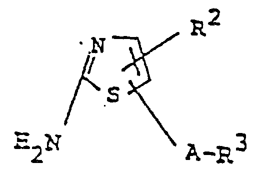 oder ihres reaktionsfähigen Derivats an der Aminogruppe oder eines Salzes derselben unter Bildung einer Verbindung der Formel 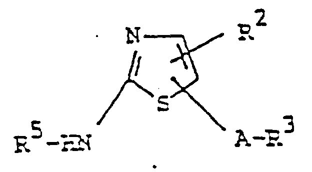 oder ihres Salzes,
worin R, R³, R⁵ und A jeweils wie oben definiert sind, oder
7) die Acylierung einer Verbindung der Formel 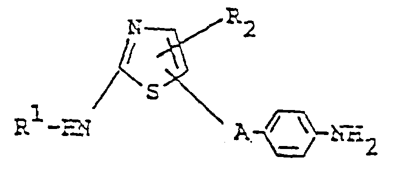 oder ihres reaktionsfähigen Derivats an der Aminogruppe oder eines Salzes derselben unter Bildung einer Verbindung der Formel 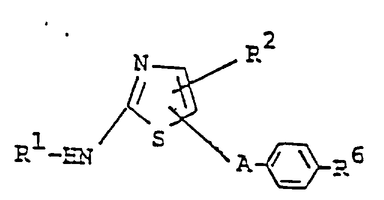 oder ihres Salzes,
worin R¹, R und A jeweils wie oben definiert sind und R⁶ für Acylamino steht, oder
8) die Umsetzung einer Verbindung der Formel 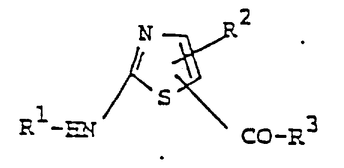 oder ihres Salzes mit einer Verbindung der Formel
H₂NOR⁴
oder ihrem Salz unter Bildung einer Verbindung der Formel 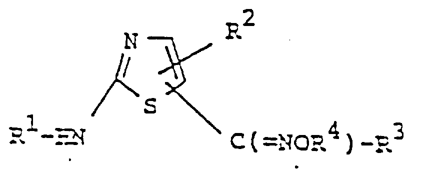 oder ihres Salzes,
worin R¹, R, R³ und R⁴ jeweils wie oben definiert sind.

## Patentansprüche (Patentansprüche für folgende(n) Vertragsstaat(en): GR)

1. Verfahren zur Herstellung einer Verbindung der Formel: 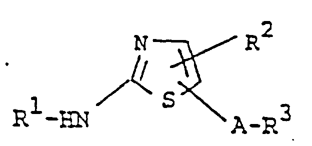 worin bedeuten:
R¹ Wasserstoff, (C₁₋₆)-Alkanoyl, (C₁₋₆)-Alkansulfonyl oder Benzoyl, die durch Halogen substituiert sein können,
R Wasserstoff, (C₁₋₆)-Alkyl, Hydroxy(C₁₋₆)-alkyl, Halogen oder Carboxy,
A -C(=NOR⁴)-, [worin R⁴ steht für (C₁₋₆)-Alkyl], 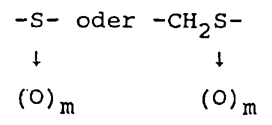 [worin m steht für die ganze Zahl 0, 1 oder 2], und
R³ Phenyl, Toluolyl, Xylyl, Cumenyl oder Naphthyl, die substituiert sein können durch Halogen, Hydroxy, (C₁₋ 6)-Alkoxy, Nitro, Amino oder Acylamino; oder Pyridyl, Pyrimidinyl, Imidazolyl, Tetrazolyl oder Thiadiazolyl, von denen jedes substituiert sein kann durch (C₁₋₆)-Alkyl, Amino, Hydroxy oder Halogen(C₁₋ ₆)-alkyl, mit der Maßgabe, daß R³ für Pyridyl, Pyrimidinyl, Imidazolyl, Tetrazolyl oder Thiadiazolyl steht, von denen jedes substituiert sein kann durch (C₁₋₆)-Alkyl, Amino, Hydroxy oder Halogen (C₁₋₆)-alkyl, wenn A 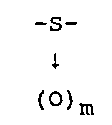 bedeutet [worin m die ganze Zahl 0, 1 oder 2 darstellt].
oder ihres Salzes, das umfaßt
1) die Deacylierung einer Verbindung der Formel 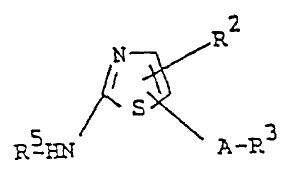 oder ihres Salzes unter Bildung einer Verbindung der Formel 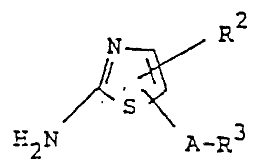 oder ihres Salzes,
worin R, R³ und A jeweils wie oben definiert sind und R⁵ für Acyl steht, das durch Halogen substituiert sein kann, oder
2) die Umsetzung einer Verbindung der Formel 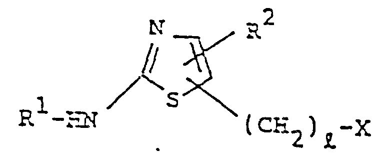 oder ihres Salzes mit einer Verbindung der Formel
R³ - SH
oder ihrem Salz unter Bildung einer Verbindung der Formel 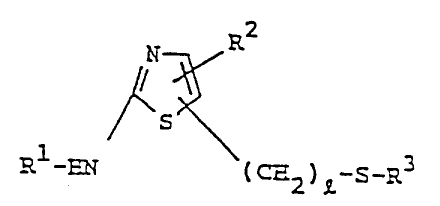 oder ihres Salzes,
worin R¹, R und R³ jeweils wie oben definiert sind und X für Halogen und 1 für 0 oder 1 stehen, oder
3) die Reduktion einer Verbindung der Formel 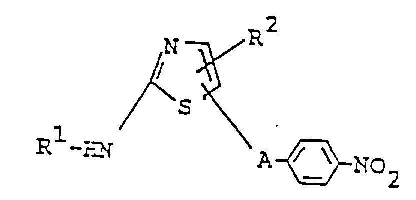 oder ihres Salzes unter Bildung einer Verbindung der Formel 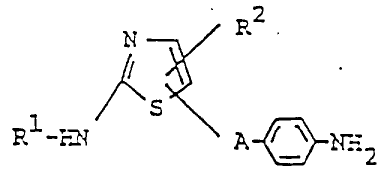 oder ihres Salzes,
worin R¹, R und A jeweils wie oben definiert sind, oder
4) die Oxidation einer Verbindung der Formel 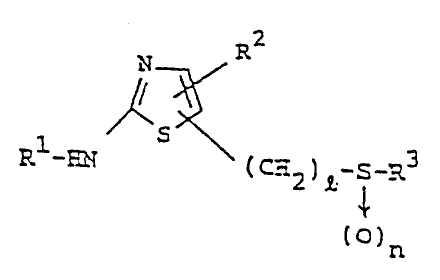 oder ihres Salzes unter Bildung einer Verbindung der Formel 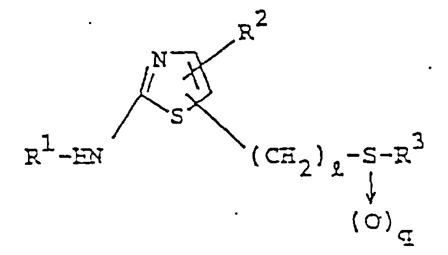 oder ihres Salzes,
worin R¹, R, R³ und 1 jeweils wie oben definiert sind, n für 0 oder 1 und q für 1 oder 2 stehen, mit der Maßgabe, daß q = 2, wenn n = 1, oder
5) die Halogenierung einer Verbindung der Formel 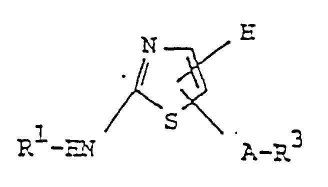 oder ihres Salzes unter Bildung einer Verbindung der Formel 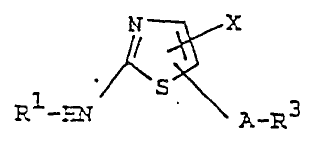 oder ihres Salzes,
worin R¹, R³, A und X jeweils wie oben definiert sind, oder
6) die Acylierung einer Verbindung der Formel 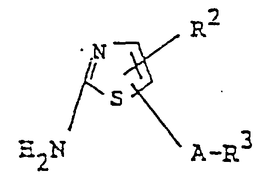 oder ihres reaktionsfähigen Derivats an der Aminogruppe oder eines Salzes derselben unter Bildung einer Verbindung der Formel 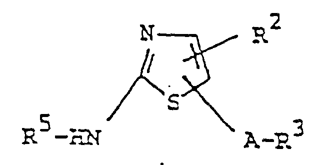 oder ihres Salzes,
worin R, R³, R⁵ und A jeweils wie oben definiert sind, oder
7) die Acylierung einer Verbindung der Formel 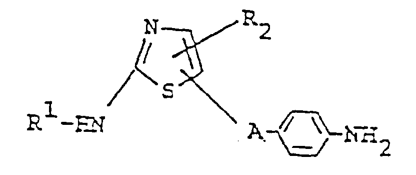 oder ihres reaktionsfähigen Derivats an der Aminogruppe oder eines Salzes derselben unter Bildung einer Verbindung der Formel 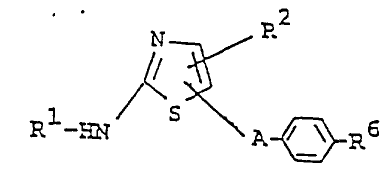 oder ihres Salzes,
worin R¹, R und A jeweils wie oben definiert sind und R⁶ für Acylamino steht, oder
8) die Umsetzung einer Verbindung der Formel 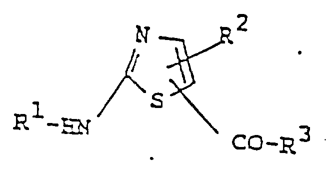 oder ihres Salzes mit einer Verbindung der Formel
H₂NOR⁴
oder ihrem Salz unter Bildung einer Verbindung der Formel 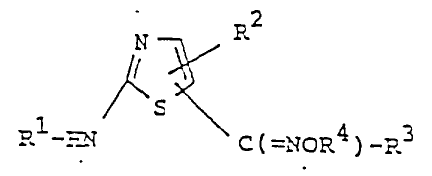 oder ihres Salzes,
worin R¹, R, R³ und R⁴ jeweils wie oben definiert sind.

2. Modifikation des Verfahrens nach Anspruch 1, dadurch gekennzeichnet, daß man eine Verbindung der Formel 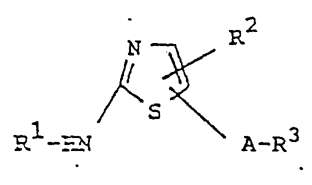 worin R¹, R, A und R³ wie in Anspruch 1 definiert sind, oder ein nicht-toxisches Salz derselben, hergestellt nach einem Verfahren, wie es in Anspruch 1 angegeben ist, in eine pharmazeutisch akzeptable Form überführt durch Mischen oder Präsentieren der genannten Verbindung mit einem pharmazeutisch akzeptablen Verdünnungsmittel oder Träger.

## Revendications (Revendications pour l'(les) Etat(s) contractant(s) suivant(s): AT, BE, CH, DE, DK, FR, GB, IT, LI, LU, NL, SE)

1. Composé de la formule : 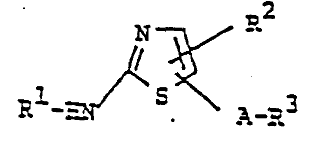 dans laquelle
R¹ est un atome d'hydrogène, un groupe alcanoyle en C₁-C₆, (alcane en C₁-C₆)sulfonyle ou benzolyle qui peut être substitué par un atome d'halogène,
R est un atome d'hydrogène, un groupe alkyle en C₁-C₆, hydroxy(alkyle en C₁-C₆), un atome d'halogène ou un groupe carboxy,
A est -C(=NOR₄)- [où R⁴ est un groupe alkyle en C₁-C₆], 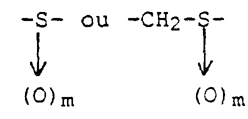 [où m est un nombre entier égal à 0, 1 ou 2], et
R³ est un groupe phényle, tuluolyle, xylyle, cuményle ou naphtyle qui peut être substitué par un atome d'halogène, un groupe hydroxy, alcoxy en C₁-C₆, nitro, amino ou acylamino; ou
pyridyle, pyrimidinyle, imidazolyle, tétrazolyle ou thiadiazolyle, dont chacun peut être substitué par un groupe alkyle en C₁-C₆, amino, hydroxy ou halo(alkyle en C₁-C₆), à condition que R³ soit un groupe pyridyle, pyrimidinyle, imidazolyle, tétrazolyle ou thiadiazolyle, dont chacun peut être substituté par un groupe alkyle en C₁-C₆, amino, hydroxy ou halo(alkyle en C₁-C₆) lorsque A est 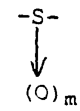 (où m est un nombre entier égal à 0, 1 ou 2),
et ses sels pharmaceutiquement acceptables.

2. Composé selon la revendication 1, dans lequel :
R¹ est un atome d'hydrogène.

3. Composé selon la revendication 2, dans lequel :
R est un atome d'hydrogène.

4. Composé selon la revendication 3, dans lequel : A est 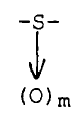 [où m est égal à 0, 1 ou 2].

5. Composé selon la revendication 4, dans lequel :
R³ est un groupe pyridyle, pyrimidinyle, imidazolyle, tétrazolyle ou thiadiazolyle.

6. Procédé pour préparer un composé de la formule : 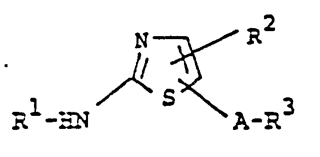 dans laquelle
R¹ est un atome d'hydrogène, un groupe alcanoyle en C₁-C₆, un groupe (alcane en C₁-C₆)sulfonyle ou benzolyle qui peut être substitué par un atome d'halogène,
R est un atome d'hydrogène, un groupe alkyle en C₁-C₆, hydroxy(alkyle en C₁-C₆), un atome d'halogène ou un groupe carboxy,
A est -C(=NOR₄)- [où R⁴ est un groupe alkyle en C₁-C₆], 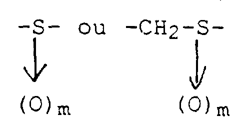 [où m est un nombre entier égal à 0, 1 ou 2], et
R³ est un groupe phényle, tuluolyle, xylyle, cuményle ou naphtyle qui peut être substitué par un atome d'halogène, un groupe hydroxy, alcoxy en C₁-C₆, nitro, amino ou acylamino; ou
pyridyle, pyrimidinyle, imidazolyle, tétrazolyle ou thiadiazolyle, dont chacun peut être substitué par un groupe alkyle en C₁-C₆, amino, hydroxy ou halo(alkyle en C₁-C₆), à condition que R³ soit un groupe pyridyle, pyrimidinyle, imidazolyle, tétrazolyle ou thiadiazolyle, dont chacun peut être substituté par un groupe alkyle en C₁-C₆, amino, hydroxy ou halo(alkyle en C₁-C₆) lorsque A est 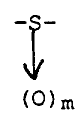 (où m est un nombre entier égal à 0, 1 ou 2),
ou son sel,
qui comprend :
(1) la désacylation d'un composé de la formule : 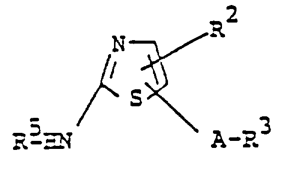 ou de son sel pour donner un composé de la formule : 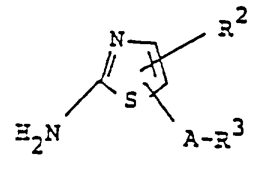 ou son sel, dans lesquelles R, R³ et A sont chacun tels que définis ci-dessus, R⁵ est un groupe acyle qui peut être subsitué par un atome d'halogène, ou
(2) la mise à réagir d'un composé de la formule : 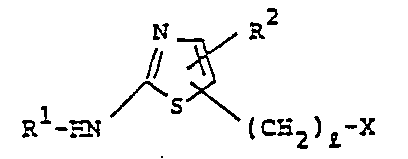 ou de son sel avec un composé de la formule :
R³ - SH
ou son sel pour donner un composé de la formule : 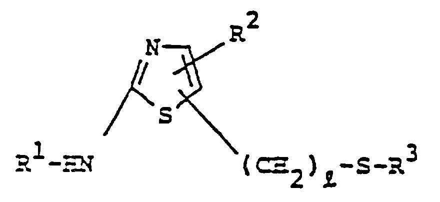
ou son sel, dans lesquelles R¹, R et R³ sont chacun tels que définis ci-dessus,
X est un atome d'halogène et ℓ est égal à 0 ou 1, ou
(3) la réduction d'un composé de la formule : 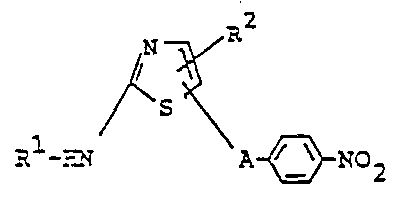 ou de son sel pour donner un composé de la formule : 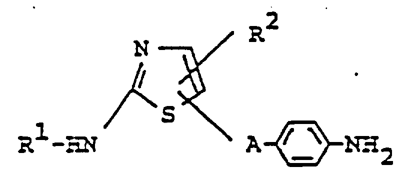 ou son sel, dans lesquelles R¹, R et A sont chacun tels que définis ci-dessus,
ou
(4) la soumission d'un composé de la formule : 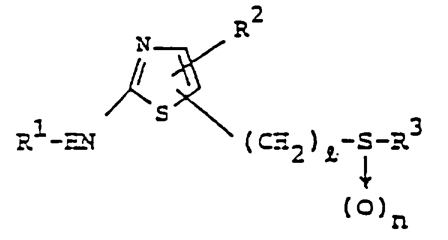 ou de son sel à une oxydation pour donner un composé de la formule : 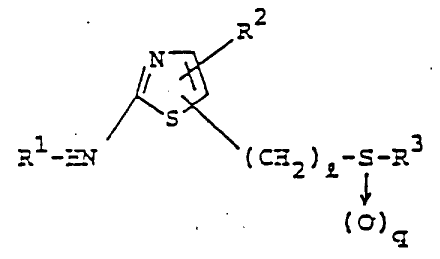 ou son sel, dans lesquelles R¹, R, R³ et ℓ sont chacun tels que définis ci-dessus, n est égal à 0 ou 1 et q à 1 ou 2, à condition que q soit 2 lorsque n est égal à 1, ou
(5) l'halogénation d'un composé de la formule : 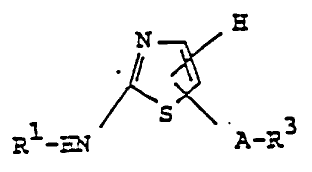 ou de son sel pour donner un composé de la formule : 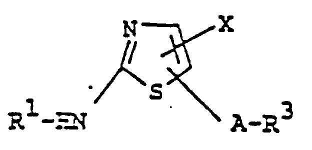 ou son sel sel, dans lesquelles R¹, R³, A et X sont chacun tels que définis ci-dessus,
ou
(6) l'acylation d'un composé de la formule : 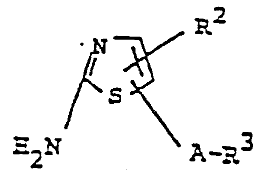 ou de son dérivé réactif au groupe amino ou d'un de ses sels pour donner un composé de la formule : 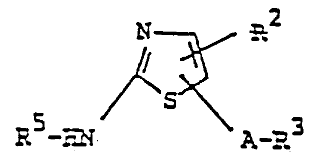 ou son sel, dans lesquelles R, R³, R⁵ et A sont chacun tels que définis ci-dessus,
ou
(7) l'acylation d'un composé de la formule : 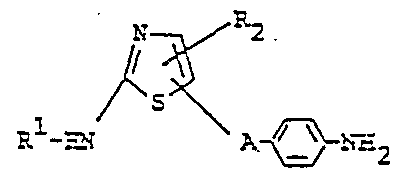 ou de son dérivé réactif au groupe amino ou d'un de ses sels pour donner un composé de la formule : 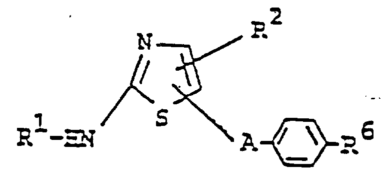 ou son sel, dans lesquelles R¹, R et A sont chacun tels que définis ci-dessus,
R⁶ est un groupe acylamino, ou
(8) la mise à réagir d'un composé de la formule : 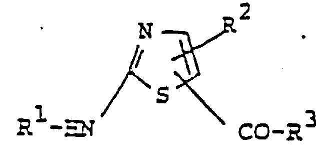 ou de son sel avec un composé de la formule :
H₂NOR⁴
ou son sel pour donner un composé de la formule : 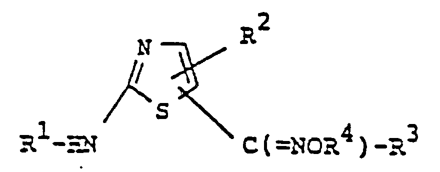 ou son sel, dans lesquelles R¹, R, R³ et R⁴ sont chacun tels que définis ci-dessus.

7. Composition pharmaceutique qui comprend, comme ingrédient actif, un composé de la revendication 1 ou un de ses sels pharmaceutiquement acceptables en mélange avec des supports pharmaceutiquement acceptables.

8. Utilisation d'un composé de la revendi-. cation 1 ou d'un de ses sels pharmaceutiquement acceptables pour la fabrication d'un médicament pour le traitement thérapeutique des rhumatismes, de la néphrite, de la thrombocytopénie, des tumeurs et des effets secondaires d'un agent anti-tumeur.

9. Composé selon la revendication 1 ou un de ses sels pharmaceutiquement acceptables pour utilisation comme médicament.

## Revendications (Revendications pour l'(les) Etat(s) contractant(s) suivant(s): ES)

1. Procédé pour préparer un composé de la formule : 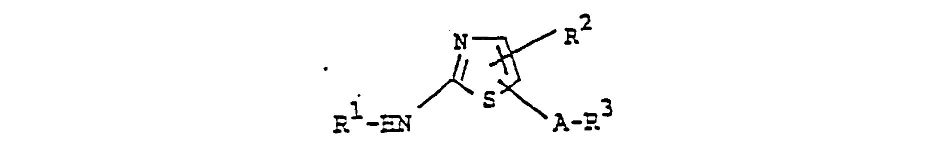 dans laquelle
R¹ est un atome d'hydrogène, un groupe alcanoyle en C₁-C₆, (alcane en C₁-C₆)sulfonyle ou benzolyle qui peut être substitué par un atome d'halogène,
R est un atome d'hydrogène, un groupe alkyle en C₁-C₆, hydroxy(alkyle en C₁-C₆), un atome d'halogène ou un groupe carboxy,
A est -C(=NOR4)- [où R⁴ est un groupe alkyle en C₁-C₆], 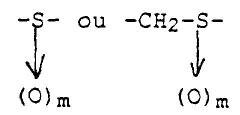 [où m est un nombre entier égal à 0, 1 ou 2], et
R³ est un groupe phényle, tuluolyle, xylyle, cuményle ou naphtyle qui peut être substitué par un atome d'halogène, un groupe hydroxy, alcoxy en C₁-C₆, nitro, amino ou acylamino; ou
pyridyle, pyrimidinyle, imidazolyle, tétrazolyle ou thiadiazolyle, dont chacun peut être substitué par un groupe alkyle en C₁-C₆, amino, hydroxy ou halo(alkyle en C₁-C₆), à condition que R³ soit un groupe pyridyle, pyrimidinyle, imidazolyle, tétrazolyle ou thiadiazolyle, dont chacun peut être substitué par un groupe alkyle en C₁-C₆, amino, hydroxy ou halo(alkyle en C₁-C₆) lorsque A est 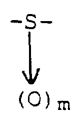 (où m est un nombre entier égal à 0, 1 ou 2),
ou son sel, qui comprend :
(1) la désacylation d'un composé de la formule : 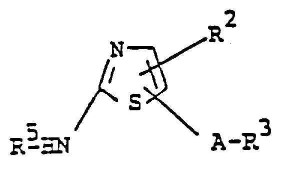 ou de son sel pour donner un composé de la formule : 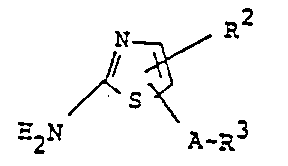 ou son sel, dans lesquelles R, R³ et A sont chacun tels que définis ci-dessus,
R⁵ est un groupe acyle qui peut être substitué par un atome d'halogène, ou
(2) la mise à réagir d'un composé de la formule : 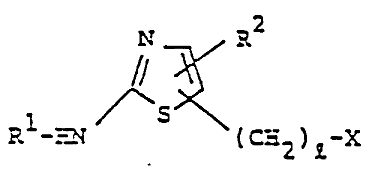 ou son sel avec un composé de la formule :
R³ - SH
ou son sel pour donner un composé de la formule : 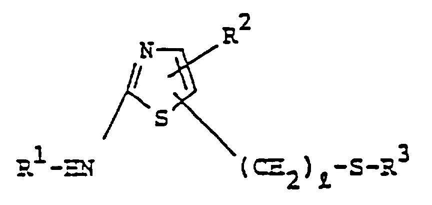 ou son sel, dans lesquelles R¹, R et R³ sont chacun tels que définis ci-dessus,
X est un atome d'halogène et ℓ est égal à 0 ou 1, ou
(3) la réduction d'un composé de la formule : 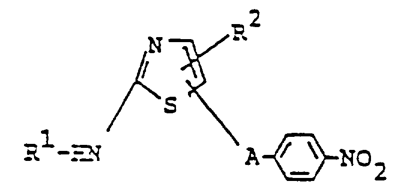 ou de son sel pour donner un composé de la formule : 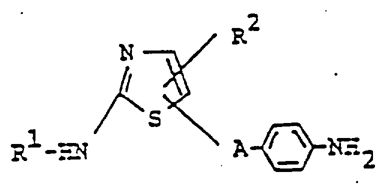 ou son sel, dans lesquelles R¹, R et A sont chacun tels que définis ci-dessus,
ou
(4) la soumission d'un composé de la formule : 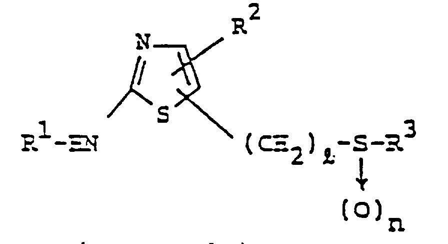 ou de son sel à une oxydation pour donner un composé de la formule : 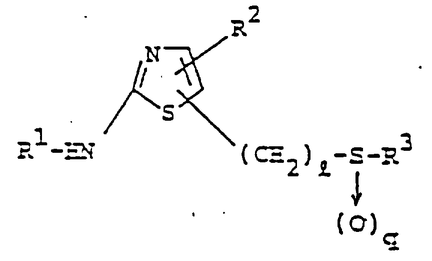 ou son sel, dans lesquelles R¹, R, R³ et ℓ sont chacun tels que définis ci-dessus,
n est égal à 0 ou 1 et q à 1 ou 2, à condition que q soit 2 lorsque n est égal à 1, ou
(5) l'halogénation d'un composé de la formule : 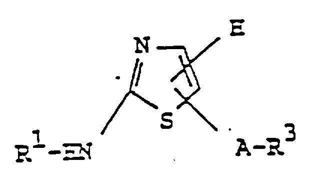 ou de son sel pour donner un composé de la formule : 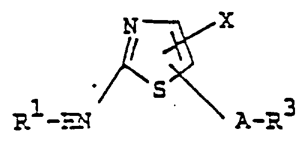 ou son sel, dans lesquelles R¹, R³, A et X sont chacun tels que définis ci-dessus,
ou
(6) l'acylation d'un composé de la formule : 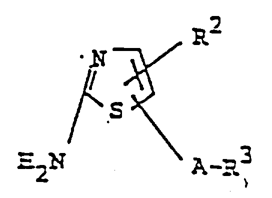 ou de son dérivé réactif au groupe amino ou d'un de ses sels pour donner un composé de la formule : 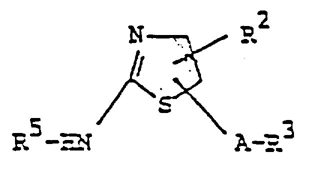 ou son sel, dans lesquelles R, R³ et R⁵ sont chacun tels que définis ci-dessus,
ou
(7) l'acylation d'un composé de la formule : 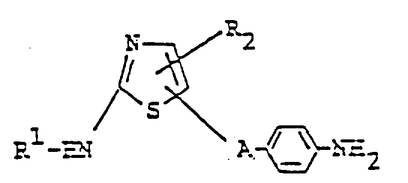 ou de son dérivé réactif au groupe amino ou d'un de ses sels pour donner un composé de la formule : 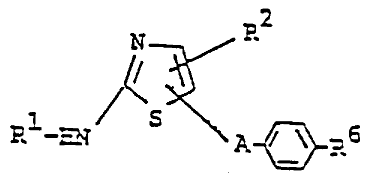 ou son sel, dans lesquelles R¹, R et A sont chacun tels que définis ci-dessus,
R⁶ est un groupe acylamino, ou
(8) la mise à réagir d'un composé de la formule : 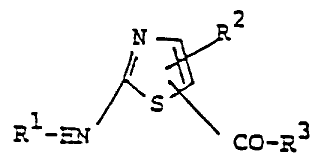 ou de son sel avec un composé de la formule :
H₂NOR⁴
ou son sel pour donner un composé de la formule : 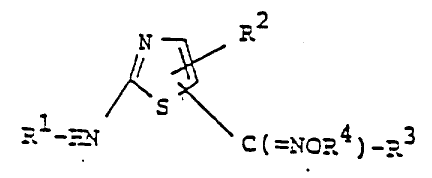 ou son sel, dans lesquelles R¹, R, R³ et R⁴ sont chacun tels que définis ci-dessus.

## Revendications (Revendications pour l'(les) Etat(s) contractant(s) suivant(s): GR)

1. Procédé pour préparer un composé de la formule : 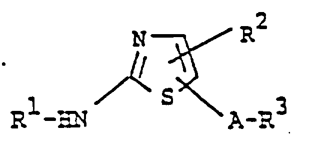 dans laquelle
R¹ est un atome d'hydrogène, un groupe alcanoyle en C₁-C₆, un groupe (alcane en C₁-C₆)sulfonyle ou benzolyle qui peut être substitué par un atome d'halogène,
R est un atome d'hydrogène, un groupe alkyle en C₁-C₆, un groupe hydroxy(alkyle en C₁-C₆), un atome d'halogène ou un groupe carboxy,
A est -C(=NOR4)- [où R⁴ est un groupe alkyle en C₁-C₆, 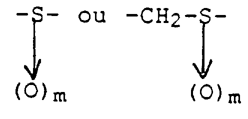 [où m est un nombre entier égal à 0, 1 ou 2], et
R³ est un groupe phényle, tuluolyle, xylyle, cuményle ou naphtyle qui peut être substitué par un atome d'halogène, un groupe hydroxy, alcoxy en C₁-C₆, nitro, amino ou acylamino; ou
pyridyle, pyrimidinyle, imidazolyle, tétrazolyle ou thiadiazolyle, dont chacun peut être substitué par un groupe alkyle en C₁-C₆, amino, hydroxy ou halo(alkyle en C₁-C₆), à condition que R³ soit un groupe pyridyle, pyrimidinyle, imidazolyle, tétrazolyle ou thiadiazolyle, dont chacun peut être substitué par un groupe alkyle en C₁-C₆, amino, hydroxy ou halo(alkyle en C₁-C₆) lorsque A est 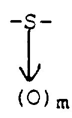 (où m est un nombre entier égal à 0, 1 ou 2),
ou son sel,
qui comprend :
(1) la désacylation d'un composé de la formule : 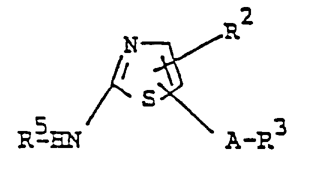 ou de son sel pour donner un composé de la formule : 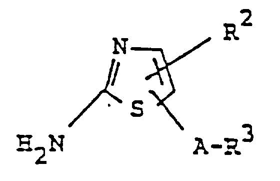 ou son sel, dans lesquelles R, R³ et R⁴ sont chacun tels que définis ci-dessus,
R⁵ est un groupe acyle qui peut être substitué par un atome d'halogène, ou
(2) la mise à réagir d'un composé de la formule : 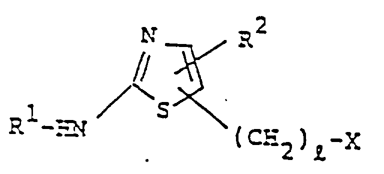 ou de son sel avec un composé de la formule :
R³ - SH
ou son sel pour donner un composé de la formule : 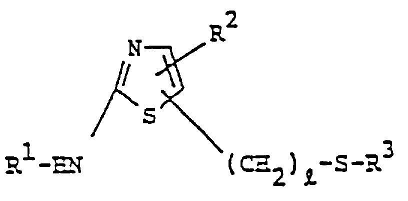 ou son sel, dans lesquelles R¹, R et R³ sont chacun tels que définis ci-dessus,
X est un atome d'halogène et ℓ est égal à 0 ou 1, ou
(3) la réduction d'un composé de la formule : 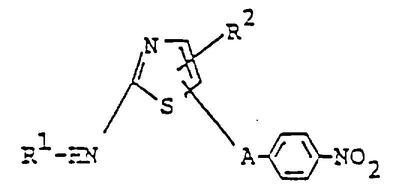 ou de son sel pour donner un composé de la formule : 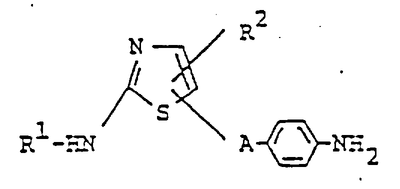 ou son sel, dans lesquelles R¹, R et A sont chacun tels que définis ci-dessus, ou
(4) la soumission d'un composé de la formule : 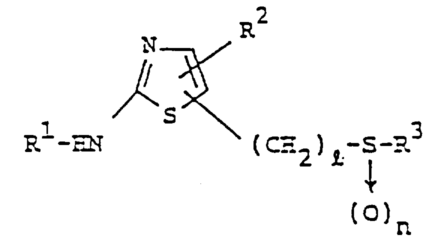 ou de son sel à une oxydation pour donner un composé de la formule : 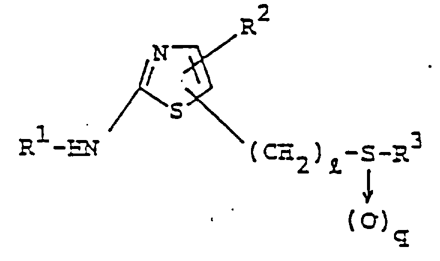 ou son sel, dans lesquelles R¹, R, R³ et ℓ sont chacun tels que définis ci-dessus,
n est égal à 0 ou 1 et q à 1 ou 2, à condition que q soit 2 lorsque n est égal à 1, ou
(5) l'halogénation d'un composé de la formule : 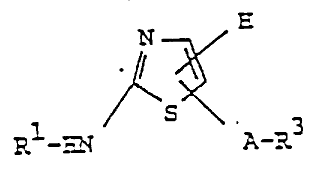 ou de son sel pour donner un composé de la formule : 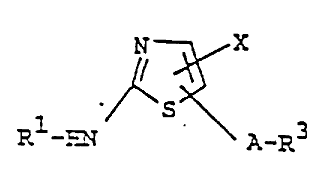 ou son sel, dans lesquelles R¹, R³, A et X sont chacun tels que définis ci-dessus,
ou
(6) l'acylation d'un composé de la formule : 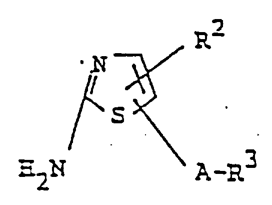 ou de son dérivé réactif au groupe amino ou d'un de ses sels pour donner un composé de la formule : 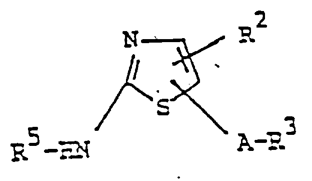 ou son sel, dans lesquelles R, R³, R⁵ et A sont chacun tels que définis ci-dessus,
ou
(7) l'acylation d'un composé de la formule : 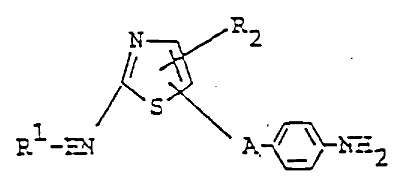 ou de son dérivé réactif au groupe amino ou d'un de ses sels pour donner un composé de la formule : 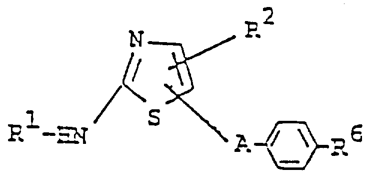 ou son sel, dans lesquelles R¹, R et A sont chacun tels que définis ci-dessus,
R⁶ est un groupe acylamino, ou
(8) la mise à réagir d'un composé de la formule : 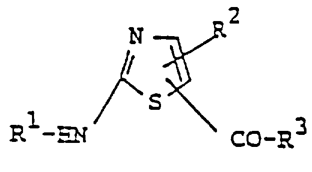 ou de son sel avec un composé de la formule :
H₂NOR⁴
ou son sel pour donner un composé de la formule : 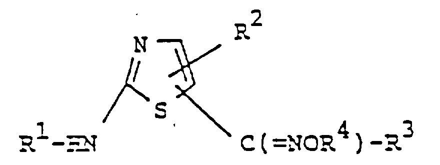 ou son sel, dans lesquelles R¹, R, R³ et R⁴ sont chacun tels que définis ci-dessus.

2. Modification du procédé revendiqué en revendication 1, qui est caractérisée en formant un composé de la formule : 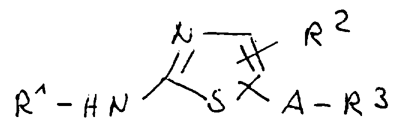 dans laquelle R¹, R, A et R³ sont tels que définis en revendication 1, ou un de ses sels non toxiques, produit par un procédé revendiqué en revendication 1, pour donner une forme pharmaceutiquement acceptable par mélange ou présentation dudit composé avec un diluant ou un support pharmaceutiquement acceptable.
